# EUROPÄISCHE PATENTSCHRIFT

(11) **EP 1 105 503 B1**
(45) Veröffentlichungstag und Bekanntmachung des Hinweises auf die Patenterteilung: **17.11.2004**
(21) Anmeldenummer: 99953535.4
(22) Anmeldetag: 16.08.1999
(51) Int. Cl.: C12N 15/60, C12N 15/62, C12N 9/88, C12P 21/02, C07K 16/40, A61K 48/00, A61P 9/10

(54) **ISOLIERTE UND GEREINIGTE HUMANE LÖSLICHE GUANYLYLCYCLASE ALPHA1/BETA1 (hsGC ALPHA1/BETA1)**
ISOLATED AND PURIFIED HUMAN SOLUBLE GUANYLYLCYCLASE ALPHA1/BETA1 (hsGCALPHA1/BETA1)
GUANYLYLCYCLASE ALPHA1/BETA1 HUMAINE SOLUBLE (hsGC ALPHA1/BETA1) ISOLEE ET PURIFIEE

(30) Priorität: 14.08.1998 DE 19837015
(43) Veröffentlichungstag der Anmeldung: 13.06.2001
(73) Patentinhaber: Vasopharm Biotech GmbH, 97082 Würzburg (DE)
(72) Erfinder: SCHMIDT, Harald, D-35392 Giessen (DE); ZABEL, Ulrike, D-97228 Rottendorf (DE); POLLER, Wolfgang, D-14612 Falkensee (DE)
(74) Vertreter: Grund, Martin, Dr.
(86) Internationale Anmeldenummer: PCT/DE1999/002601
(87) Internationale Veröffentlichungsnummer: WO 2000/009711

(56) Entgegenhaltungen:
- BEHRENDS ET AL.: "A variant of the alpha 2 subunit of soluble guanylyl cyclase contains an insert homologous to a region within adenylyl cyclases and functions as a dominant negative protein" THE JOURNAL OF BIOLOGICAL CHEMISTRY, Bd. 270, Nr. 36, 8. September 1995 (1995-09-08), Seiten 21109-21113, XP002132769
- GANSEMANS ET AL.: "Human soluble guanylate cyclase large subunit mRNA, alpha3-like" EMBL DATABASE ACC NO: U58855, 9. Januar 1998 (1998-01-09), XP002132770
- GIUILI ET AL.: "Molecular cloning of the cDNAs coding for the two subunits of soluble guanylyl cyclase from human brain" FEBS LETTERS, Bd. 304, Juni 1992 (1992-06), Seiten 83-88, XP002132771 in der Anmeldung erwähnt
- GUPTA ET AL.: "Expression and purification of soluble, active heterodimeric guanylyl cyclase from baculovirus" PROTEIN EXPRESSION AND PURIFICATION, Bd. 10, Nr. 3, August 1997 (1997-08), Seiten 325-330, XP000877369
- OLSON ET AL.: "Selective guanylyl cyclase inhibitor reverses nitric oxide-induced vasorelaxation" HYPERTENSION, Bd. 29, 1997, Seiten 254-261, XP002132772
- ZABEL ET AL.: "Human soluble guanylate cyclase: functional expression and revised isoenzyme family" BIOCHEMICAL JOURNAL, Bd. 335, Oktober 1998 (1998-10), Seiten 51-57, XP002132773
- ZABEL ET AL.: "Homodimerization of soluble guanylyl cyclase subunits" THE JOURNAL OF BIOLOGICAL CHEMISTRY, Bd. 274, Nr. 26, 25. Juni 1999 (1999-06-25), Seiten 18149-18152, XP002132774

## Beschreibung

### Das technische Gebiet der Erfindung

Die vorliegende Erfindung betrifft die Expression der cDNA-Klone für di Untereinheiten α1 (hsGCα1) und β1 (hsGCβ1) der humanen löslichen Guanylylcyclase und die nachfolgende Aufreinigung des aktiven Enzyms und dessen Anwendung, die medizinische Anwendung der Expression dieser Klone durch Gentransfer, sowie Antikörper gegen aus der Sequenz abgeleitete Peptide und deren Anwendung.

### Diskussion des Standes der Technik

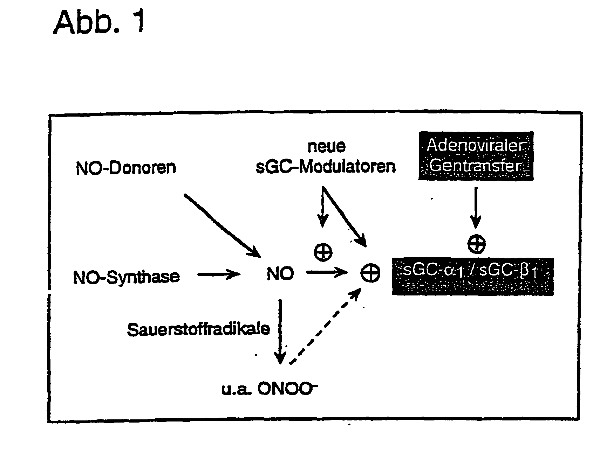

Das körpereigene NO/cGMP Signalsystem vermittelt wichtige Funktionen wie Vasodilatation, Hemmung der Thrombocytenaggregation, Neurotransmission und Immunabwehr und ist auch an der Entstehung verschiedener Erkrankungszustände wie Ischämie-Reperfusion und Entzündungsschäden beteiligt (Schmidt und Walter, 1994). Daher ist das NO/cGMP-System seit langem ein wichtiger Ansatzpunkt für die Entwicklung neuer Pharmaka zur Therapie der koronaren Herzkrankheit, Thromboseneigung, Herzinsuffizienz, Angina pectoris, des kardial bedingten Lungenödems, hypertensiver Krise, Entzündungs- und Infarktzuständen. Bisher werden hier verschiedene sogenannte NO-Donoren wie Glyceroltrinitrat u.a. eingesetzt, die NO freisetzen, dadurch endogenes NO ersetzen und lösliche Guanylylcyclase (sGC) aktivieren (Abb. 1). sGC bildet cGMP, das seinerseits über verschiedene intrazelluläre Rezeptorenzyme die Wirkungen des NO/cGMP-Signalweges übermittelt. Die Gabe von NO-Donoren hat zwei Einschränkungen: 1.) führt die häufige Gabe von NO-Donoren zur Toleranz beim Patienten, d.h. Wirkungsverlust gegenüber einer erneuten Gabe. 2.) reagiert NO mit O₂⁻ zu zytotoxischem und bzgl. der Stimulation von sGC weniger effektivem Peroxynitrit. Daher wäre eine direkte Aktivierung der sGC durch neue, nicht NO-haltige Pharmaka für die Therapie oder den Gentransfer von sGC wünschenswert. Nicht-NO-freisetzenden sGC-Stimulatoren fehlen potentiell Toleranz und NO-Toxifizierung. Mit "YC-1" (= 3-(5'-Hydroxymethyl-2'-furyl)-1-benzylindazol) wurde inzwischen ein erster solcher, nicht NO-freisetzender Aktivator von sGC in Thrombozyten beschrieben (Ko et al., 1994; Wu et al., 1995). YC-1 aktiviert auch gereinigte sGC aus Rinderlunge und potenziert die NO-Aktivierung (Friebe et al., 1996). Die Wirkung auf humane sGC ist nicht untersucht. Für ein Pharmaka-Screening steht die humane Isoform der sGC jedoch bisher nicht zur Verfügung.

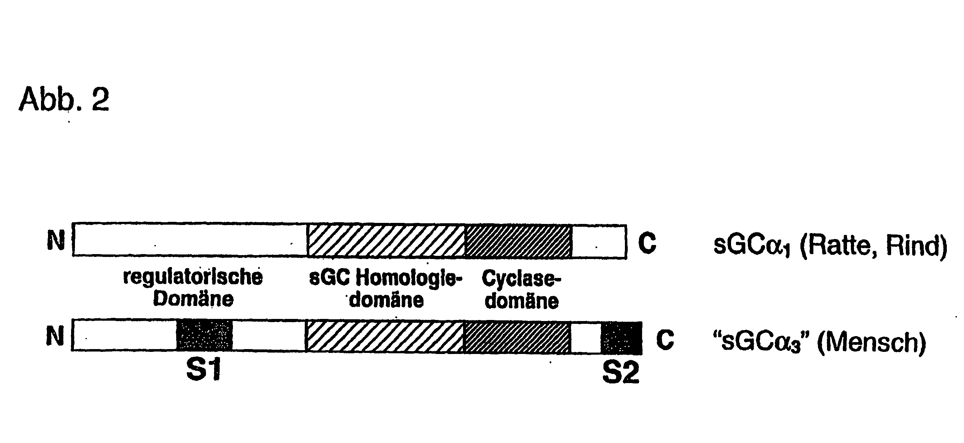

Der NO-Rezeptor sGC besteht aus zwei Untereinheiten, **α** und β, die zusammen ein enzymatisch aktives Heterodimer büden. In der Literatur sind jedoch drei α- und drei β-Untereinheiten, allerdings aus verschiedenen Spezies, beschrieben worden. Am besten untersucht sind die bovine α1/β1 und die Ratten-α1/β1-Isoform, die insbesondere für die kardiovaskuläre Forschung große Bedeutung haben Gupta G. et al., haben die Expression und Reinigung der Guanylylcyclase α1/β1 aus Ratte beschrieben (Prot. Ex.and Pw., 10, 325-330,1997). Ein humanes Homolog der sGC aus Rind und Ratte (z.B. sGCα1/sGCβ1-Heterodimere) war bis vor kurzem nicht bekannt Publiziert sind cDNA-Sequenzen, die einer humanen sGC-Isoform α3 und β3 entsprechen sollten (Giuili et al., 1992). Während sGCβ3 sehr hohe Homologie zu sGCβ1 (Rind/Ratte) aufwies, enthielt die sGCα3-Sequenz zwei begrenzte Bereiche ohne Homologie zu sGCα1 (Rind/Ratte), hier als S1 und S2 bezeichnet (Abb. 2). Es war darüber hinaus nicht bekannt, ob sGCα3 und sGCβ3 eine funktionelle heterodimere sGC konstituieren können, und welche Rolle dabei die Bereiche S1 und S2 spielen. Auch keine andere humane sGC-Unbereinheit wurde bisher als Protein exprimiert Kürzlich wurden in der GenBank eine als hsGCα1 bezeichnete Sequenz veröffentlicht (Accesion-Nr. U58855), der die Sequenzunterschiede zu sGCα1 von Rind und Ratte in den Bereichen S1 und S2 fehlen. Außerdem wurde in der GenBank kürzlich auch eine alternatives Spleißprodukt zur hsGCβ3 veröffentlicht (Accesion-Nr. AF020340), das dort als alternativ gespleißte Form der hsGCβ1 bezeichnet wird. Die physiologische Bedeutung dieser Spleißvariante von hsGCβ1/3 ist jedoch völlig unklar. Damit stellt sich die Frage, welche dieser Isoformen für welche physiologischen Funktionen in welchen Zelltypen verantwortlich ist.

Derzeit gibt es außerdem keine Antikörper gegen die humane sGCα1/β1, die monospezifisch sind, gegen die humanen Sequenzen gerichtet und deren Eignung für den Immunoblot mit humanen Geweben nachgewiesen worden ist. Bisher veröffentlichte Peptidantikörper zeigen diese Eigenschaften nur zum Teil: Harteneck et al. und Guthmann et al. verwenden eine Peptidsequenz (VYKVETVGDKYMTVSGLP), die in Guanylylcyclasen recht hohe Konservierung zeigen, so daß eine Kreuzreaktion auch mit partikulären Guanylylcyclasen (z.B. GC-C) erwartet werden kann. Guthmann et al. verwendet eine zu hsGCβ1 identische Peptidsequenz (YGPEVWEDIKKEA) und eine bis auf zwei Aminosäureaustausche identische Peptidsequenz zu hsGCα1 (KKDVEEANANFLGKASGID). Allerdings ist die Funktion dieser Antikörper im Immunoblot nur für *angereinigte* hsGC aus humanen Thrombozyten gezeigt Zusätzlich erkennen die Antiseren gegen hsGCα1 noch ein zweites, unspezißsches Produkt Humbert et al. und Koesling et al. verwenden eine Peptidsequenz (SRKNTGTEETEQDEN) aus Rindes-sGCβ1, die teilweise (Aminosäuren 1-10) mit dem hier verwendeten Peptid (Aminosäuren 13-22) für humane sGCβ1 überlappt und dort identisch ist, dessen C-Terminus (Aminosäuren 11-15) jedoch deutlich verschieden von der humanen Sequenz ist. Das Antiserum gegen dieses Peptid wurde jedoch nicht an humanem Protein getestet, sondern nur für die Immunpräzipitation der bovinen sGC verwendet.

Damit standen von der für die kardiovaskuläre Forschung wichtigen humanen Isoform α1/β1 weder *natives* Protein, noch *rekombinantes* Protein, ein einstufiges Reinigungsprotokoll oder spezifische Antikörper zur Verfügung. Auch ein Ansatz zur Gentherapie (z.B. mit Adenoviren o. ä.) ist bisher nicht beschrieben.

### Das der Erfindung zugrundeliegende technische Problem

Um neue Pharmaka oder Gentransfertechniken für die kardiovaskuläre Therapie finden zu können, die weder zur Toleranz beim Patienten führen noch zytotoxisches Peroxynitrit bilden, ist eine NO-unabhängige Aktivierung von humaner sGC α1/β1 ein erfolgversprechender Ansatz. Um solche Pharmaka zu finden, ist ein Massenscreening nach geeigneten Wirkstoffen erforderlich. Ein solches Pharmakascreening nach spezifischen Aktivatoren oder Inhibitoren ist im Tierversuch zu teuer und wegen Speziesunterschieden, möglichen Nebenwirkungen und Effekten auf andere Isoformen nicht sinnvoll. Zellkultursysteme haben den Nachteil, daß nur mit erheblichem Mehraufwand festgestellt werden kann, wo Substanzen innerhalb der Signalkaskaden ihre Wirkungen entfalten. Außerdem ist die Zellkultur kostenintensiv und empfindlich. Die Aufreinigung eines Proteins aus tierischen Geweben ist aufwendig bei geringerer Ausbeute. Vor allem aber sind die Ergebnisse eines solchen Screenings aufgrund der Speziesunterschiede nur bedingt übertragbar. Hier stellt sich insbesondere die Frage nach der Bedeutung der Isoformen hsGCα1 und hsGCα3 sowie die Frage, ob hsGCβ3 tatsächlich einer humanen hsGCβ1 entspricht. Dies ist wichtig, um das richtige Zielprotein für ein pharmakologisches Screening oder eine Gentherapie auszuwählen. Für ein optimales Screening müßte das humane Homolog des Proteins sGC α1/β1 in großen Mengen aufgereinigt und kostengünstig zur Verfügung stehen. Eine Aufreinigung großer Mengen humanen nativen sGC-α1/β1-Proteins aus entsprechend humanem Gewebe ist nicht möglich. Daher sind für das Pharmakascreening andere Methoden nötig. Zusätzlich gibt es keine geeigneten Antikörper zum Nachweis der humanen sGCα1/β1, deren Eignung für diagnostische Zwecke z.B. im normalen Immunoblot, ELISA, RIA, EIA o.ä., gezeigt ist Auch gibt es bisher keinen Ansatz für die therapeutisch genutzte, künstliche Expression von hsGC durch Gentransfer im Menschen.

Der Erfindung liegt somit die Aufgabe zugrunde, isolierte und gereinigte humane sGCα1/β1 sowie ein Verfahren zu ihrer Herstellung und Reinigung bereitzustellen. Eine weitere Aufgabe der Erfindung ist es, Antikörper gegen die humane sGCα1/β1 bereitzastellen. Außerdem ist es Aufgabe der Erfindung, Expressionsvektoren auf Basis von Adenoviren bereitzustellen, welche die cDNA der humanen sGCα1/β1 enthalten. Schließlich ist es Aufgabe der Erfindung, die humane sGCα1/β1 gereinigt und in handhabbaren Mengen bereitzustellen für ein Pharmakascreening nach Modulatoren, Inhibitoren und Aktivatoren der humanen sGCα1/β1.

### Die Lösung des technischen Problems

Die Lösung des vorstehenden technischen Problems wird erreicht durch die in den Patentansprüchen gekennzeichneten Ausführungsformen und die nachstehenden Erläuterungen der Erfindung.

Gegenstand der Erfindung ist somit eine isolierte und zur apparenten Homogenität gereinigte humane lösliche Guanylylcyclase α1/β1 (hsGCα1/β1), wobei hsGC21 identifiziert ist durch SEQ ID NO: 2 und hsGCβ1 identifiziert ist durch SEQ ID NO: 4.

Ein weiterer Gegenstand der Erfindung ist ein Verfahren zur Herstellung der der humanen löslichen Guanylylcyclase α1/β1, umfassend die Expression eines die DNA-Sequenz für die Untereinheiten hsGCα1 und HsGCβ1 enthaltenden Expressionsvektores in prokaryontischen oder eukaryontischen Wirtszellen und Gewinnung der Guanylylcyclase.

In einer bevorzugten Ausführungsform des erfindungsgemäßen Verfahrens zur Herstellung der humanen löslichen Guanylylcyclase α1/β1 umfaßt der Schritt der Gewinnung der Guanylylcyclase α1/β1 die Lyse der Zellen, die Affinitätschromatographie des Zell-Lysats und die anschließende Elution der Guanylylcyclase α1/β1.

In einer weiter bevorzugten Ausführungsform des erfindungsgemäßen Verfahrens zur Herstellung der Untereinheit α1/β1 der humanen löslichen Guanylylcyclase enthält der Expressionsvektor zusätzlich mindestens eine DNA-Sequenz für eine Domäne zur spezifischen Affinitätschromatographie (Affinitätstag) mit anhängender Proteaseschnittstelle.

Ein weiterer Gegenstand der Erfindung ist ein Verfahren zur Herstellung der humanen löslichen Guanylylcyclase α1/β1 (hsGCα1/β1), umfassend die getrennte Expression eines die DNA-Sequenz für hsGCα1 oder hsGCβ1 enthaltenden Expressionsvektors in prokaryontischen oder eukaryontischen Wirtszellen, Gewinnung der Untereinheiten und Kombinieren der Untereinheiten hsGCα1 und hsGCβ1 zur dimeren Guanylylcyclase α1/β1 (hsGCα1/β1).

Eine weitere Ausführungsform des erfindungsgemäßen Verfahrens zur Herstellung der humanen löslichen Guanylylcyclase α1/β1 (hsGCα1/β1) umfaßt die Koexpression der DNA-Sequenzen für hsGCα1 und hsGCβ1 in prokaryontischen oder eukaryontischen Wirtszellen, die Lyse der hsGCα1 und hsGCβ1 enthaltenden Zellen, die Affinitätschromatographie und die anschließende Elution der löslichen Guanylylcyclase.

Ein weiterer Gegenstand der Erfindung ist Verwendung einer Nukleinsäuresequenz, die die Untereinheit hsGCα1 (SEQ ID NO: 2) und hsGCβ1 (SEQ ID NO: 4) der humanen löslichen Guanylylcyclase α1/β1 kodiert, als Wirkstoff zur Herstellung eines Arzneimittels zur somatischen Gentherapie, insbesondere zur Prävention und Therapie von Atherosklerose und ihren Folgeerkrankungen, von Restenose, Ischämie (Infarkt), peripheren arteriellen Verschlufskrankheiten und arterieller Hypertonie sowie zur Prävention bei Patienten mit Risikofaktoren für Atherosklerose, transitorische ischämische Attacken; zerebrale Ischämie, Schlaganfall (Apoplex), Koronare Herzkrankheit, Zustand nach koronarer Bypass-Operation, Carotisstenose, zur Verstärkung einer Therapie mit sGC-Aktivatoren, sGC-sensitivierenden Substanzen, Stickstoffmonoxid (NO)-Donoren oder Phosphodieserase-Hemmstoffen, Herzinsuffizienz und Leberfunktionsstörungen.

In einer besonders bevorzugten Ausführungsform werden Adenovektoren mit hsGCα1- und hsGCβ1-cDNA für die somatische Gentherapie eingesetzt. jedoch können auch andere Vektorsysteme für eine gentherapeutische medizinische Nutzung verwendet werden.

In einer bevorzugten Ausführungsform werden adenovirale Ad5 und andere geeignete Vektoren zur Prävention und Therapie der vorstehend-genannten Erkrankungen verwendet, welche die Nukleinsäuresequenz für humane lösliche Guanylylcyclase α1 (hsGCα1) und humane lösliche Guanylylcyclase β1 (hsGCβ1) enthalten. Ebenso kann zum somatischen Gentransfer ein Gemisch von zwei Vektoren eingesetzt werden, bei dem ein Vektor die Nukleinsäuresequenz für humane lösliche Guanylylcyclase α1 (hsGCα1) und der zweite Vektor die Nukleinsäuresequenz für humane lösliche Guanylylcyclase β1 (hsGCβ1) enthält. Besonders bevorzugt ist der somatische Gentransfer in Endothelzellen, glatte Blutgefäßmuskelzellen, Neointimazellen, Fibroblasten und anderen Blutgefäßzellen sowie in die korpuskulären Blutbestandteile (Thrombozyten, Leukozyten und andere) und Leber.

Die erfindungsgemäß beschriebenen Methoden zum Gentransfer können auch für den Gentransfer für die humane lösliche Guanylylcydase α2 (GenBank: x63282) und für das humane Homolog zur löslichen Guanylylcyclase β2 (aus Ratte; GenBank: m57507) und für andere humane lösliche Guanylylcyclasen angewendet werden.

Ein weiterer Gegenstand der Erfindung betrifft monospezifische Antikörper gegen humane löslich Guanylylcyclase α1/β1 (hsGCα1/β1), erhältlich durch Immunisieren eines Säuger mit hsGCα1/β1, und Isolieren der Antikörper.

### Die Abbildungen zeigen:

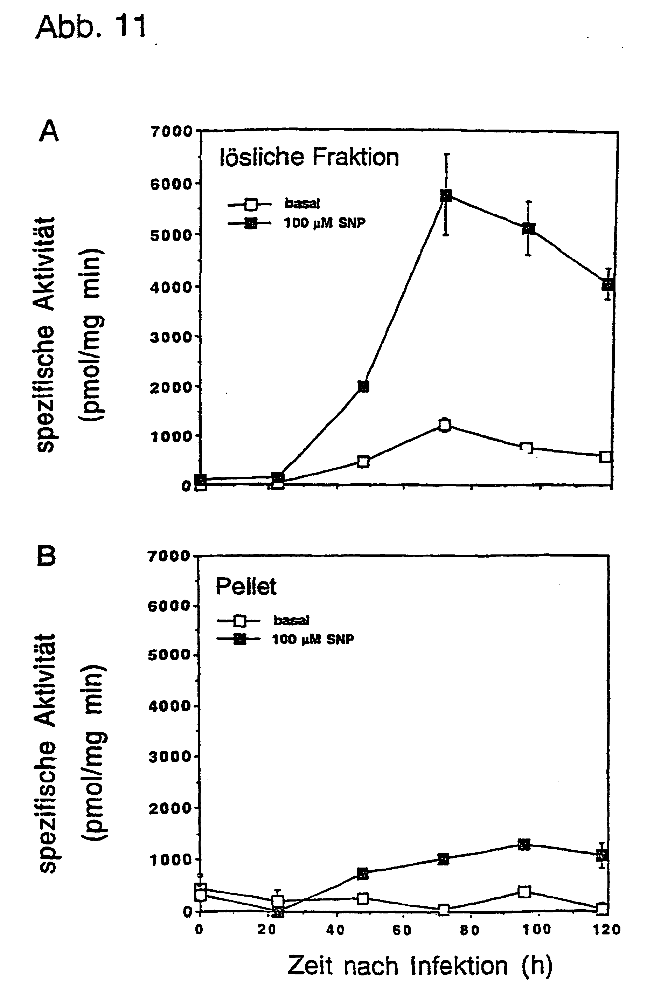

Abbildung 1 zeigt die verschiedenen Modulationsmöglichkeiten der löslichen Guanylylcyclase (sGC-α1/sGC-β1). Die normale Aktivierung erfolgt über NO-Synthase (NOS) und NO (Stickstoffmonoxid). NO reagiert aber mit Sauerstoffradikalen u.a. zu Peroxynitrit (ONOO⁻), das zytotoxisch ist und sGC nur schwach aktiviert NO kann zusätzlich über NO-Donoren wie Glycerolnitrat oder Natriumnitoprussid freigesetzt werden. Durch sGC-Modulatoren (z.B. YC-1), kann sGC direkt aktiviert werden oder aber der Aktivierungseffekt von NO auf sGC wird durch diese Modulatoren verstärkt. Durch Gentransfer (z.B. mittels adenoviraler Vektoren) läßt sich zusätzlich eine Überexpression der sGC erreichen, bzw. ein pathologisch bedingt zu geringer Expressionsspiegel ausgleichen. Es könnten auch adenovirale (oder andere) Vektoren mit sGC-Mutanten eingesetzt werden, die eine höhere Basalaktivität zeigen. Dadurch ließe sich unabhängig von NOS, NO, NO-Donoren oder sGC-Modulatoren dauerhaft ein erhöhter cGMP-Spiegel erreichen.
Abbildung 2 zeigt einen schematischen Vergleich der sGCα1-Untereinheit von Rind bzw. Ratte mit der veröffentlichten Sequenz des als "sGCα3" bezeichneten humanen cDNA-Klons (Giuili et al., 1992). Die Balken stellen dabei das Protein dar. Dabei bezeichnet "N" den N-Terminus, "C" den C-Terminus. Markiert sind mit verschiedenen Schraffuren die funktionellen Abschnitte "regulatorische Domäne", "sGC-Homologiedomäne" und "Cyclasedomäne" dieser Proteine. Schwarz markiert sind die beiden Bereiche "S1" und "S2", für die sich keine homologen Bereiche in den Proteinen der sGCα1 von Rind und Ratte finden.

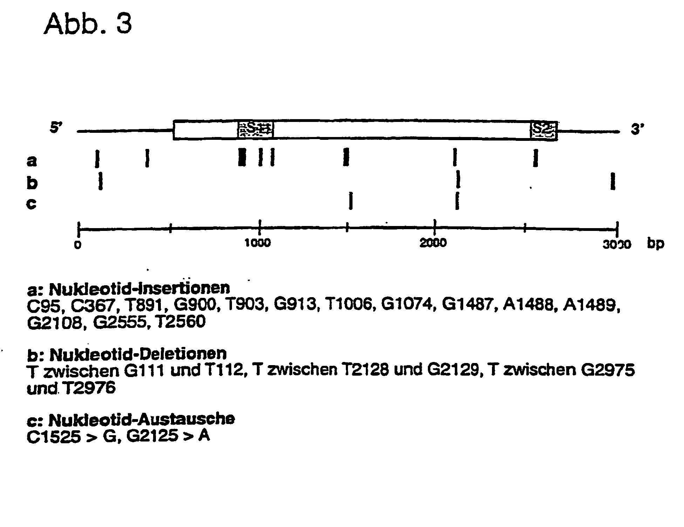

Abbildung 3 zeigt eine schematische Darstellung des humanen sGCα3-Klons mit den veröffentlichten Sequenzierfehlern (Giuili et al., 1992). Oben ist die cDNA gezeigt: Der Balken stellt den kodierenden Bereich der cDNA dar, die Striche links und rechts davon zeigen die untranslatierten Bereiche am 5'- und 3'-Ende. S1 und S2 stellen die Bereiche fehlender Homologie zu den Isoformen sGCα1 von Rind und Ratte dar (vgL Abb. 2). Darunter sind die Positionen der Sequenzierfehler eingezeichnet: Zeile a zeigt die Nukleotidinsertionen, Zeile b die Deletionen und Zeile c die Austausche. Darunter ist ein Maßstab in Basenpaaren (bp) gezeigt. Ganz unten sind für jede der drei Zeilen a, b und c die Sequenzierfehler aufgerührt: Der Buchstabe gibt dabei die Art der betroffenen Base an, die Zahl ihre Position in der cDNA.

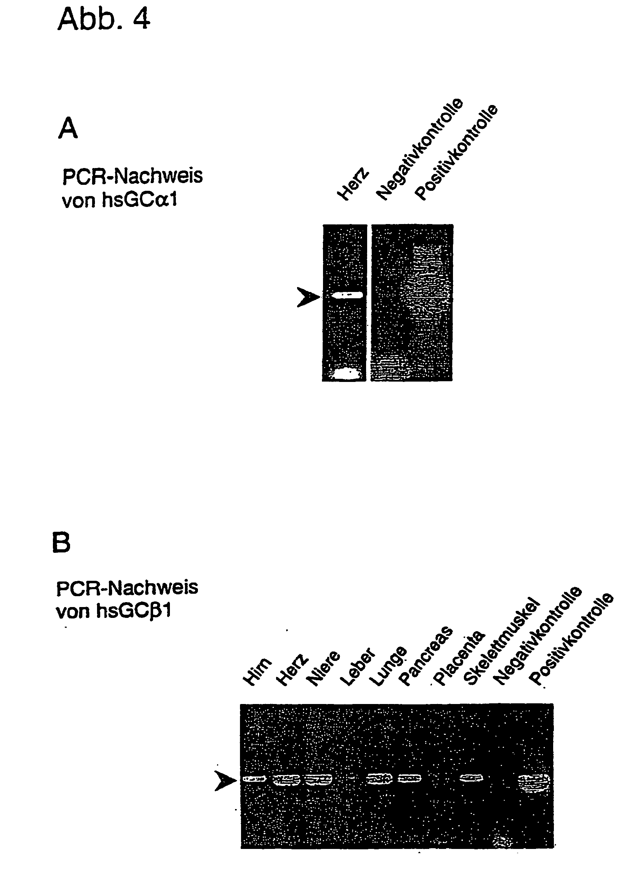

Abbildung 4 zeigt den Nachweis der Expression von humaner sGCα1 (A) und sGCβ1 (B) in humanen Geweben mittel PCR in cDNA-Banken. Abgebildet ist ein Foto eines ethidiumbromidgefärbten Agarosegels mit aufgetrennten PCR-Produkten unter UV-Licht. Der Pfeil links zeigt das spezifische Produkt an. Ganz unten in den Fotos sind außerdem die PCR-Primer zu sehen. Oben ist jeweils das Gewebe angegeben, aus dem die cDNA-Bank hergestellt wurde. Bei der Negativkontrolle wurde keine cDNA zugegeben, bei der Positivkontrolle wurde Plasmid mit der cDNA der hsGCα1 zugegeben.

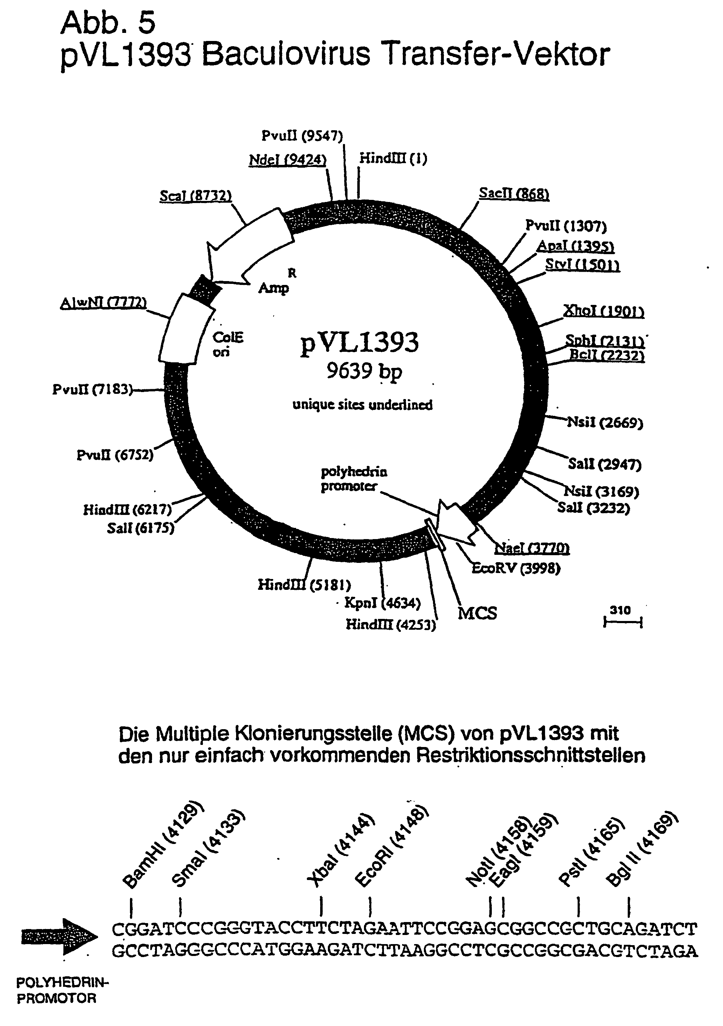

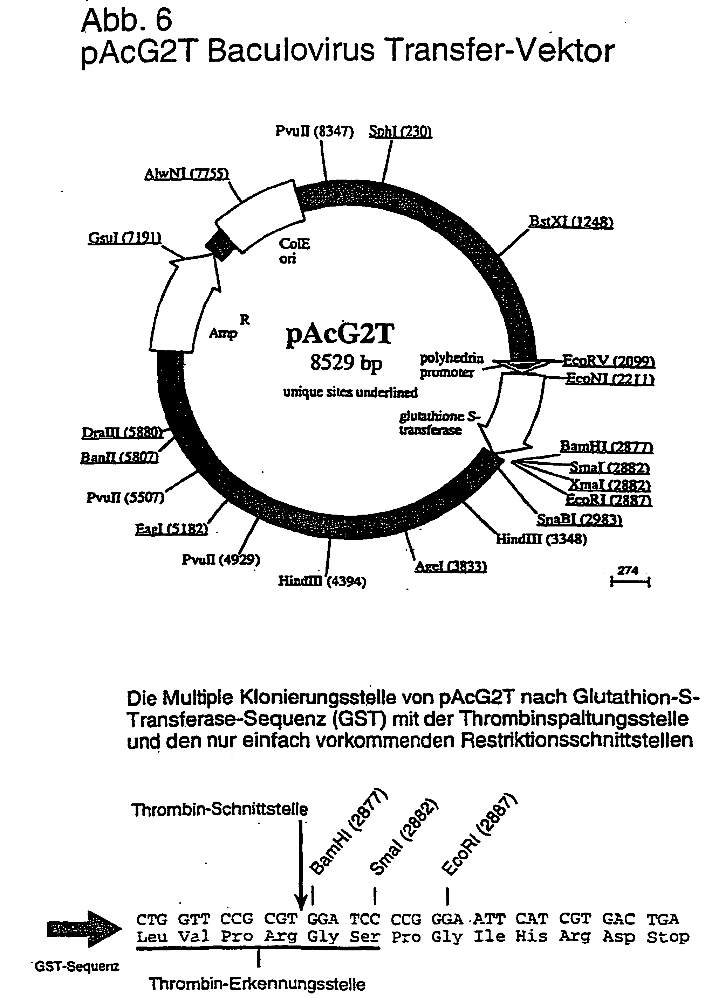

Abbildungen 5 und 6 zeigen die Baculovirus-Transfervektoren pVL1393 bzw. pAcG2T (jeweils ohne die hsGC-cDNA), die für die Konstruktion der rekombinanten Baculoviren zur Expression der humanen sGCα1/β1 in Sf9-Zellen verwendet wurden. Oben ist jeweils das ringförmige Plasmid mit den Restriktionsschnittstellen (Kurzname und Position in Basenpaaren), dem Gen für Ampicilin-Resistenz (Amp^{R}), dem "origin of replication" (ColE ori), dem Polyhedrinpromotor, der Glutathion-S-Transferase-Sequenz (nur Abb. 6) und der Multiplen Klonierungsstelle (MCS) dargestellt. Abbildung 5 zeigt unten die Multiple Klonierungstelle mit den Restriktionsschnittstellen, die nur einmal im Plasmid vorkommen. Abbildung 6 zeigt unten die Multiple Klonierungstelle mit den Restriktionsschnittstellen, die nur einmal im Plasmid vorkommen, sowie einer Thrombinschnittstelle.

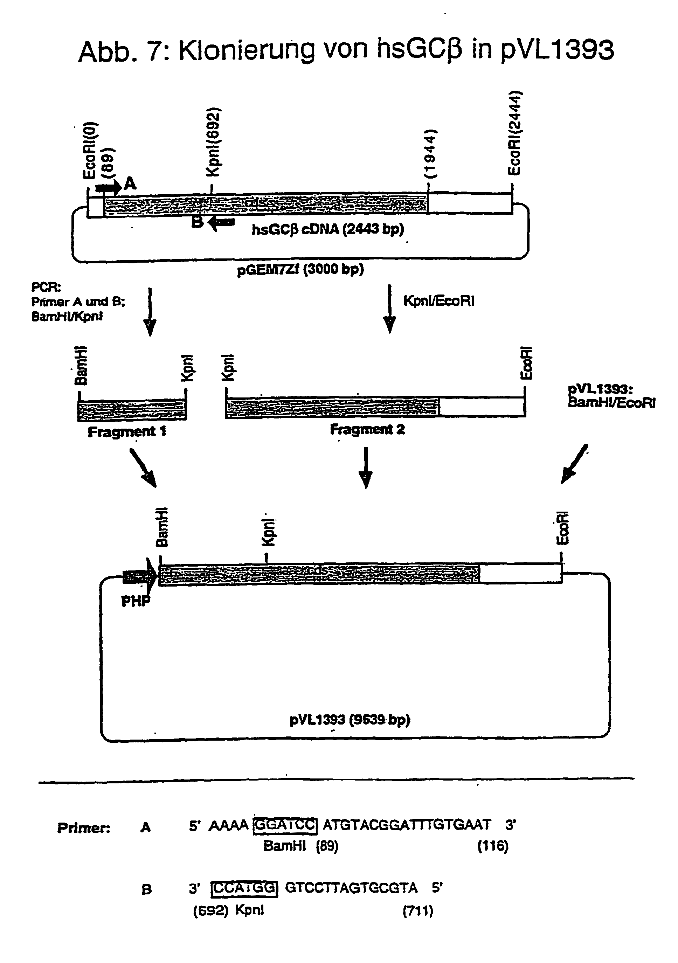

Abbildung 7 zeigt die Konstruktion des Plasmids hsGCβ1-pVL1393 (ohne GST-Tag) mit der hsGCβ1-cDNA, das benutzt wurde, um durch homologe Rekombination den gentechnisch veränderten, hsGCβ1-exprimierenden Baculovirus zu erhalten. Die Vorgehensweise für das Plasmid pAcG2T-hsGCβ1 (mit GST-Tag = Glutathion-S-Transfetase-cDNA aus *Schistosoma japonicum*) ist identisch. Mittels einer PCR wurde mit den Primern A (Basen 89-116 der hsGCβ1-cDNA + BamHI-Schnittstelle am 5'-Ende) und B (Basen 692-711 der hsGCβ1-cDNA [Gegenstrang] mit natürlicher Schnittstelle KpnI) ein Fragment hergestellt, das an seinem 5'-Ende zusätzlich eine BamHI-Schnittstelle trägt Durch diese zusätzliche Schnittstelle konnte Fragment 1 (PCR-Fragment mit neuer BamHI-Schnittsbelle) und Fragment 2 (hsGCβ1-cDNA von Schnittstelle KpnI bis Schnittstelle EcoRI) zusammen in die Schnittstellen BamHI und EcoRI des Plasmids pVL1393 inseriert werden, wodurch eine komplette hsGCβ1-cDNA unter der Kontrolle des Polyhedrinpromotors (PHP) zu liegen kommt

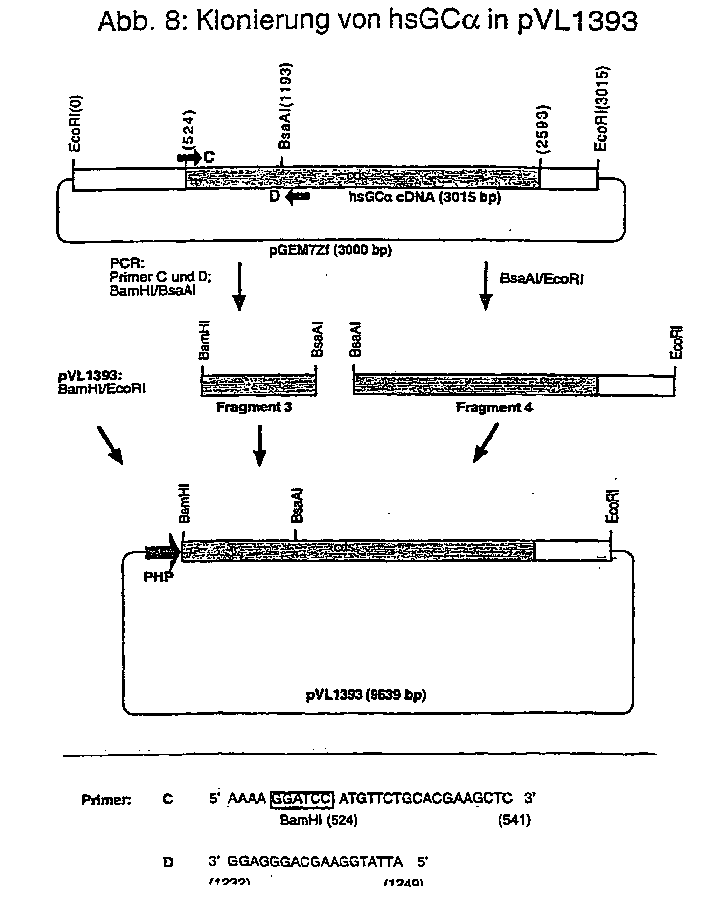

Abbildung 8 zeigt die Konstruktion des Plasmids hsGCα1-pVL1393 (ohne GST-Tag) mit der hsGCα1-cDNA, das benutzt wurde, um durch homologe Rekombination den gentechnisch veränderten, hsGCα1-exprimierenden Baculovirus zu erhalten. Die Vorgehensweise für das Plasmid pAcG2T-hsGCα1 (mit GST-Tag = Glutathion-S-Transferase-cDNA aus *Schistosoma japonicum*) ist identisch. Mittels einer PCR wurde mit den Primern C (Basen 524-541 der hsGCα1-cDNA + BamHI-Schnittstelle am 5'-Ende) und D (Basen 1232-1249 der hsGCα1-cDNA [Gegenstrang]) ein Fragment hergestellt, das an seinem 5'-Ende zusätzlich eine BamHI-Schnittstelle und das in dem Fragment eine natürliche BsaAI-Schnittstelle trägt. Durch diese zusätzliche Schnittstelle konnte das BsaAI-gespaltene Fragment 3 (PCR-Fragment mit neuer BamHI-Schnittstelle bis BsaAI) und Fragment 4 (hsGCα1-cDNA von Schnittstelle BsaAI bis Schnittstelle EcoRI) zusammen in die Schnittstellen DamHI und EcoRI des Plasmids pVL1393 inseriert werden, wodurch eine komplette hsGCα1-cDNA unter der Kontrolle des Polyhedrinpromotors (PHP) zu liegen kommt.

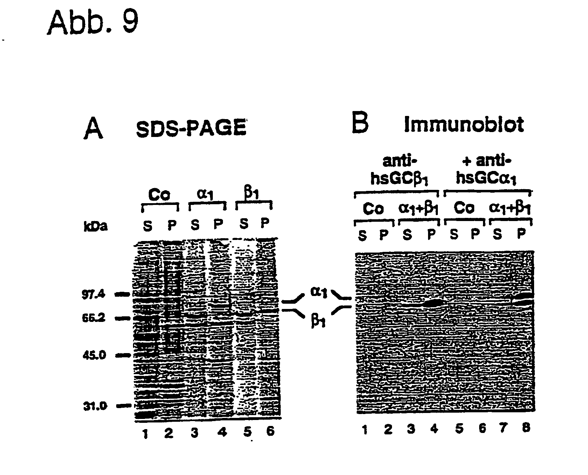

Abbildung 9 zeigt den Nachweis der Expression von hsGCα1/β1 in Sf9-Zellen, die mit den oben beschriebenen gentechnisch veränderten Viren (mit hsGCα1- oder hsGCβ1 -cDNA; beide ohne GST-Tag = Glutathion-S-Transferase-cDNA aus *Schistosoma japonicum*) infiziert wurden. Links (A) ist ein Coomassie-gefärbtes 10 %iges SDS-Polyacrylamidgel gezeigt, auf dem durch Zentrirugation (20.000 x g) in Pellet (P) und Überstand (S) aufgetrenntes Zellhomogenat aufgtragen wurde. "Co" bezeichnet dabei die Kontrolle mit uninfizierten Sf9-Zellen. "α1" bezeichnet Sf9-Zellen, die mit Viren mit der hsGCα1-cDNA infiziert wurden, während "β1" Sf9-Zellen bezeichnet, die mit Viren mit der hsGCβ1-cDNA infiziert wurden. Die Position der hsGCα1 und hsGCβ1 im Gel ist jeweils gekennzeichnet (α1 bzw β1). Rechts davon ist ein Immunoblot mit Überstand (S) und Pellet (P) des Zellhomogenates von Sf9-Zellen gezeigt, die entweder uninfiziert (Co) oder mit hsGCα1- und hsGCβ1-Baculovirus koinfiziert wurden (α1+β1). Im Immunoblot wurden zuerst die oben beschriebenen Peptidantikörpern gegen hsGCβ1 (antihsGCβ1) eingesetzt (Abb. 9B, Spuren 1-4). Anschließend wurde der Blot nochmals mit den Peptidantiköpern gegen hsGCα1 (anti-hsGCα1) entwickelt (Abb. 9B, Spuren 5-8), wodurch hier auch die hsGCβ1-Banden sichtbar sind.

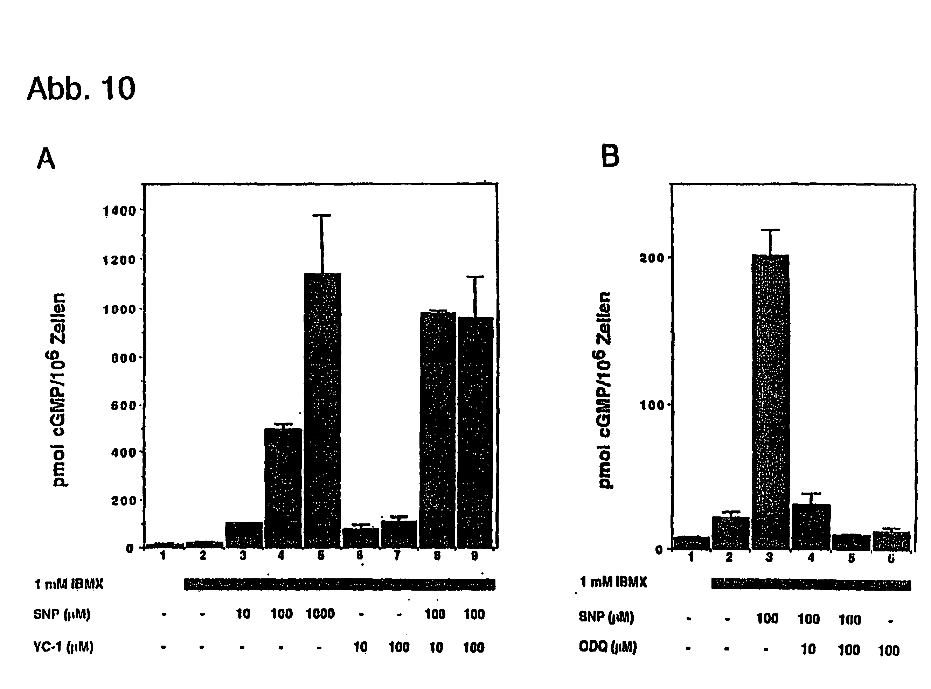

Abbildung 10 zeigt den Nachweis der Guanylylcyclase-Aktivität (Bildung von cGMP aus GTP) in intakten Sf9-Zellen, die mit den hier beschriebenen gentechnisch veränderten Baculoviren mit hsGCα1- und hsGCβ1-cDNA (beide ohne GST-Tag) koinfiziert werden. Gezeigt ist jeweils der Gehalt an cGMP in pmol pro 10⁶ Zellen bei unterschiedlicher Behandlung mit Pharmaka: Unten sind jeweils die zugesetzten bei unterschiedlicher Behandlung mit Pharmaka: Unten sind jeweils die zugesetzten Pharmaka angegeben. Probe 1 ist in beiden Teilgrafiken A und B unbehandelt. Bei den übrigen Proben wurde jeweils 1 mM IBMX (3-Isobutyl-1-methylxanthin) zugesetzt (oberste Zeile: schwarzer Querbalken). In der mittleren Zeile ist die in der jeweiligen Probe zugesetzte Menge an SNP in µM angegeben. Die unterste Zeile zeigt die Menge an zugesetztem YC-1 (A, links) oder ODQ (B, rechts) in µM.
   YC-1 = 3-(5'-Hydroxymethyl-2'-furyl)-1-benzylindazol;
   ODQ = 1H-[1,2,4]oxadiazolo[4,3,-a]chinoxalin-1-on;
   SNP = Nitroprussidnatrium;
   cGMP = zyklisches 3',5'-Guanosinmonophosphat
Abbildung 11 zeigt den Nachweis der Guanylylcyclase-Aktivität (Bildung von cGMP aus GTP) im Homogenat von Sf9-Zellen, die mit den hier beschriebenen gentechnisch veränderten Baculoviren mit hsGCα1- und hsGCβ1-cDNA (beide ohne GST-Tag) koinfiziert wurden. Oben (A) ist die lösliche Zellfraktion (Überstand nach Zentrifugation bei 20000 x g) eingesetzt worden, unten (B) das zugehörige Pellet Gezeigt ist jeweils die Menge an gebildetem cGMP in pmol pro mg Protein und pro Minute in Homogenat von Zellen, die zu verschiedenen Zeitpunkten (Angaben in Stunden) nach der Infektion mit den Baculoviren geerntet wurden. Dabei wurde die cGMP-Bildung mit (schwarze Kästchen) und ohne (weiße Kästchen) Zusatz von 100 µM SNP gemessen.

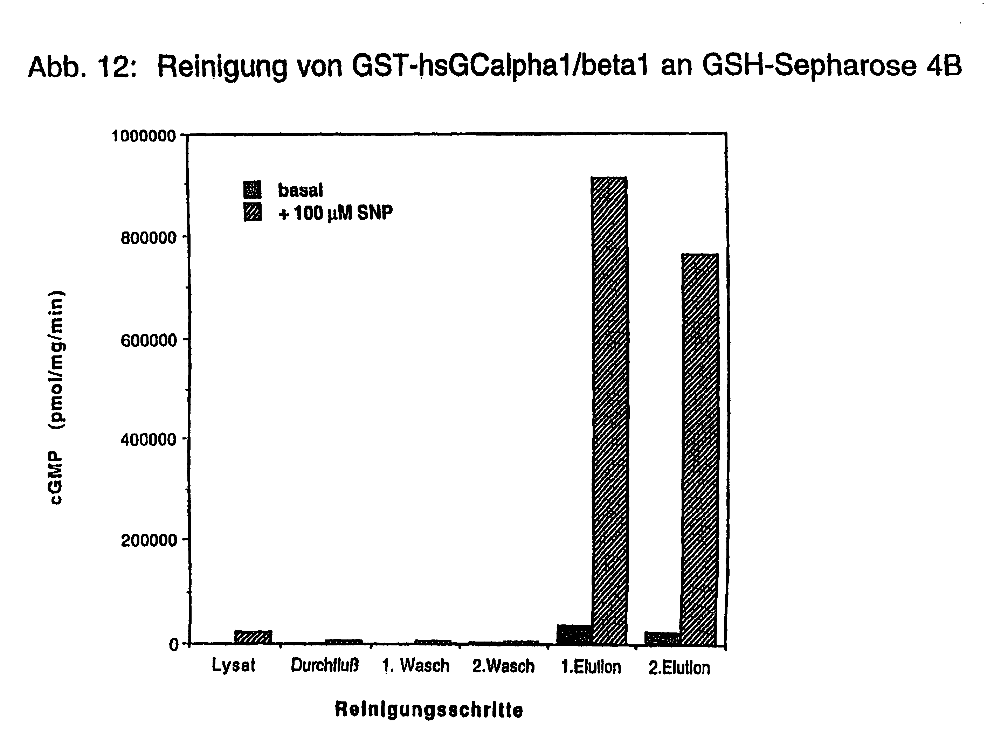

Abbildung 12 zeigt den Nachweis der Guanylylcyclase-Aktivität (Bildung von cGMP aus GTP) in Sf9-Zellen, die mit den hier beschriebenen gentechnisch veränderten Baculoviren mit hsGCβ1-cDNA (ohne GST-Tag) und hsGCα1-cDNA (mit GST-Tag = Glutathion-S-Transferase-cDNA aus *Schistosoma japonicum*) koinfiziert wurden. Gezeigt ist die Bildung von cGMP in pmol pro mg Protein und pro Minute während des Reinigungsprozesses (Affinitätschromatographie an Glutathion-Sepharose 4B). Gemessen wurde jeweils im Lysat (nach Abzentrifugieren des unlöslichen Anteils bei 20000 x g), der Überstand nach Bindung der hsGC an die Glutathion-Sepharose 4B (Durchfluß), die beiden Überstände vom Waschen der hsGC-gebundenen Glutathion-Sepharose 4B (1. und 2. Wasch) sowie der Überstand nach Elution der hsGC mit reduziertem Glutathion (1. und 2. Elution). Unterschieden wurde dabei die cGMP-Bildung ohne (schwarze Kästchen, "basal") und mit (graue Kästchen, "+100 µM SNP") Zusatz von 100 µM SNP.

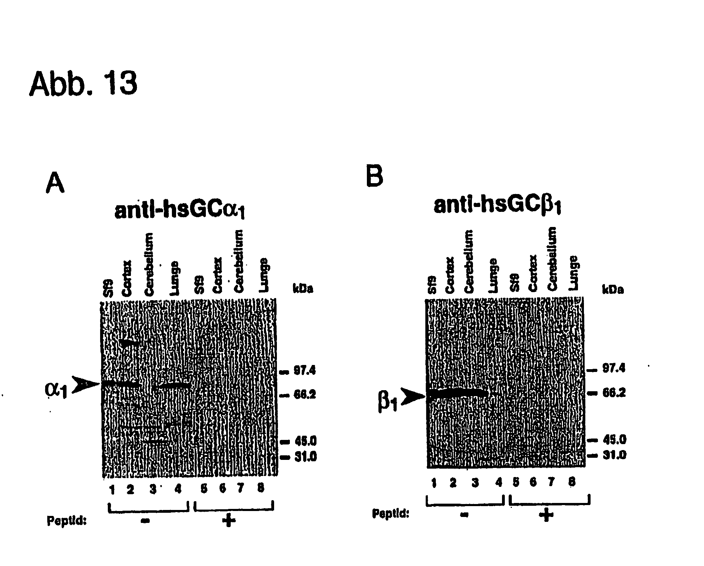

Abbildung 13 zeigt den Nachweis der natürlichen Expression von hsGCα1 und hsGCβ1 in verschiedenen humanen Geweben im Immunoblot.
   Links (A) wurden die oben beschriebenen Peptidantikörper gegen hsGCα1 (antihsGCα1) eingesetzt, rechts (B) die Peptidantiköper gegen hsGCβ1 (anti-hsGCβ1). Dabei wurde als Neagtivkontrolle jeweils in der rechten Hälfte das Peptid zugesetzt, gegen das die Antikörper gerichtet sind (Peptid: +), während in der jeweils linken Hälfte kein Peptid zugegeben wurde (Peptid: -). Auf ein 8 % iges Polyacrylamidgel wurden jeweils SDS-Extrakte von rhsGC α1- (in Teilabb. A) oder rhsGCβ1-überexprimierenden Sf9-Zellen (in Teilabb. B) (Sf9), von humanem Großhirencortex (Cortex), von humanem Cerebellum (Cerebellum) und von humaner Lunge (Lunge) aufgetragen. Die spezifischen Banden von hsGCα1 (α1) und hsGCβ1 (β1) sind jeweils mit einem Pfeil gekennzeichnet.

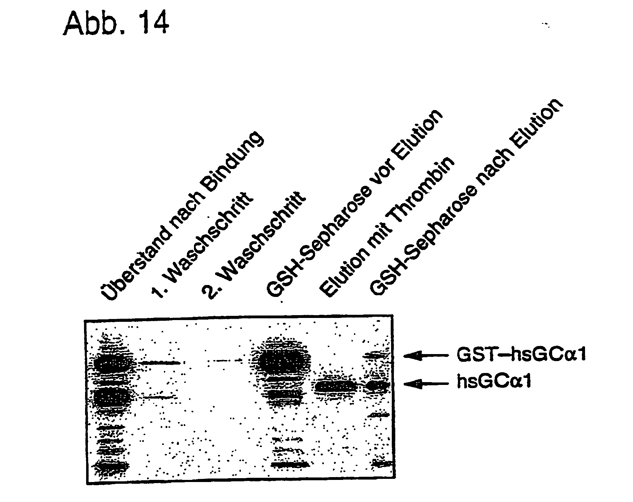

Abbildung 14 zeigt im Immunoblot den Nachweis der Aufreinigung von hsGCα1 (als hsGCα1/hsGCβ1-Dimere) aus Sf9-Zellen, die mit den hier beschriebenen gentechnisch veränderten Baculoviren mit hsGCα1-cDNA mit GST-Tag (= Glutathion-S-Transferase-cDNA aus *Schistosoma japonicum*) und hsGCβ1-cDNA (ohne GST-Tag) koinfiziert wurden. Zellysat wurde mit Glutathion-Sepharose-4B inkubiert, und der Überstand nach der Bindung aufgetragen (Überstand nach Bindung). Die Sepharose wurde zweimal gewaschen, und jeweils der Überstand dieses Waschpuffers aufgetragen (1. und 2. Waschschritt). Dann wurde eluiert durch Abspalten des hsGCα1-Proteins vom GST-Tag mit Thrombin und ein Aliquot des Überstandes aufgetragen ("Elution mit Thrombin"). Danach wurde die Glutathion-Sepharose 4B in SDS-Stoppuffer aufgenommen und ein Aliqout aufgetragen (GSH-Sepharose nach Elution). Zusätzlich wurde Glutathion-Sepharose 4B mit gebundenem hsGCα1 ohne vorherige Thrombin-Elution in SDS-Stoppuffer aufgenommen und ein Aliquot aufgetragen (GSH-Sepharose vor Elution). Der Immunoblot wurde mit den hier beschriebenen affinitätsgereinigten Peptidantikörpern gegen den C-Terminus der hsGCα1 entwickelt. Die Pfeile rechts zeigen die spezifischen Banden von hsGCα1 mit dem anhängenden GST-Tag (GSThsGCα1) und hsGCα1 ohne GST-Tag (hsGCα1).

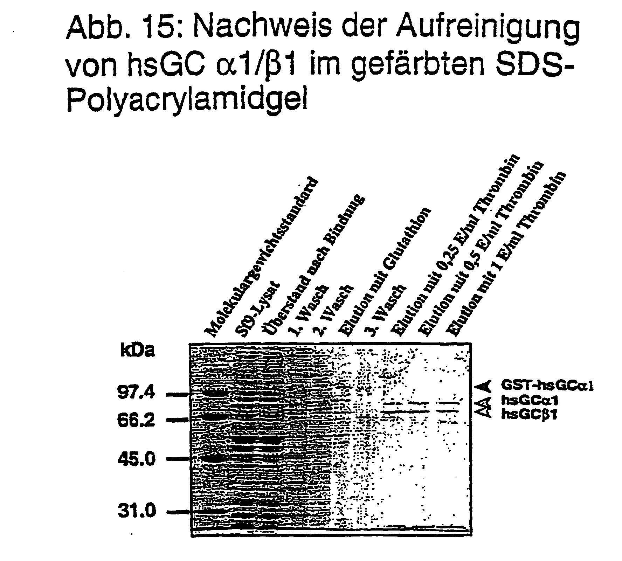

Abbildung 15 zeigt im Coomassie-Brilliant-Blue-R250-gefärbten SDS-Polyacrylamidgel den Nachweis der Aufreinigung von hsGCα1/β1 aus Sf9-Zellen, die mit den hier beschreibenen rekombinanten Baculoviren mit hsGCα1-cDNA mit GST-Tag (= Glutathion-S-Transferase-cDNA aus Schistosoma japonicum) und hsGCβ1-cDNA ohne GST-Tag koinfiziert wurden. Zellysat dieser infizierten Sf9-Zellen (Lysat) wurde mit Glutathion-Sepharose 4B inkubiert, und der Überstand nach der Bindung aufgetragen (Überstand nach Bindung). Die Glutathion-Sepharose 4B wurde zweimal gewaschen, und jeweils der Überstand des Waschpuffers aufgetragen (1. und 2. Wasch). In einer Probe wurde das gebundene GSThsGCα1/β1 durch Inkubation mit reduziertem Glutathion eluiert und aufgetragen (Elution mit Glutathion). Bei den anderen Proben wurde die Glutathionsepharose mit dem Puffer für die Thrombinspaltung - ohne Thrombin - gewaschen und der Überstand dieses Puffers aufgetragen (3. Wasch). Dann wurde das hsGCα1/β1-Dimer eluiert durch Inkubation mit verschiedenen Mengen Thrombin und die Eluate aufgetragen (Elution mit 0,25-1 E/ml Thrombin) [E = Einheiten]. Verwendet wurden jeweils gleiche relative Mengen jeder Probe. Rechts sind die bei den verschiedenen Elutionsmethoden sichtbaren Banden gekennzeichnet: GST-hsGCα1 = hsGCα1 mit GST-Tag; hsGCα1=hsGCα1 ohne GST-Tag; hsGCβ1= hsGCβ1 ohne GST-Tag. Links sind Molekulargewichtsstandards auf das Gel aufgetragen, deren Größe ganz links in kDa angegeben ist

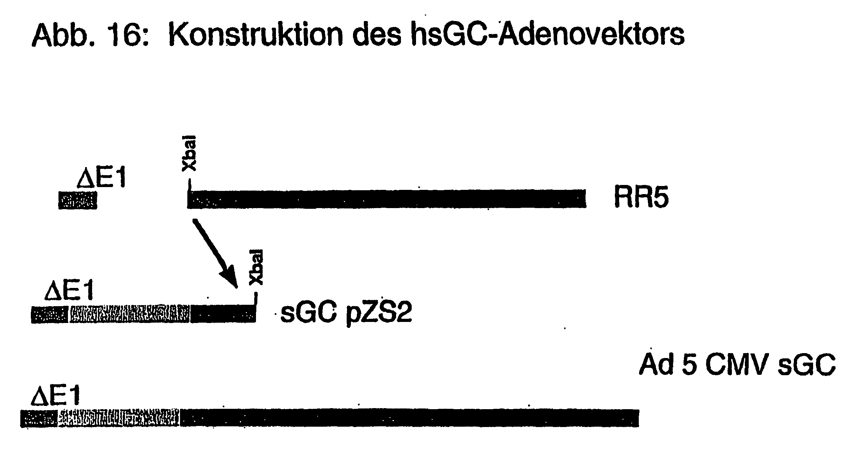

Abbildung 16 zeigt die Konstruktion der rekombinanten adenoviralen hsGC-Vektoren. Die cDNAs für die hsGCα1 und hsGCβ1 (graue Balken) wurden in das adenovirale Transferplasmid pZS2 inseriert, das eine Deletion in der Adenovirus-E1-Region (ÆE1) enthält und eine in diesem Plasmid einmaligen XbaI-Schnittstelle. Dies ergab die Plasmide hsGCα1-pZS2 bzw. hsGCβ1-pZS2
   Mit dem Restriktionsenzym XbaI geschnittenes hsGCα1-pZS2 und hsGCβ1-pZS2 (mittlerer Balken, dargestellt als "sGCpZS2") wurde in die XbaI-Schnittstelle des langen Armes (oberer Balken, "RR5") von Ad5 ligiert. Dies ergab die Adenovektoren Ad5CMVhsGCα1 bzw. Ad5CMVhsGCβ1 (unterer Balken, "Ad 5 CMV sGC"), in dem die sGC-cDNAs unter der Kontrolle des CMV-Promotors und -Enhancers liegen (CMV = Cytomegalovirus)

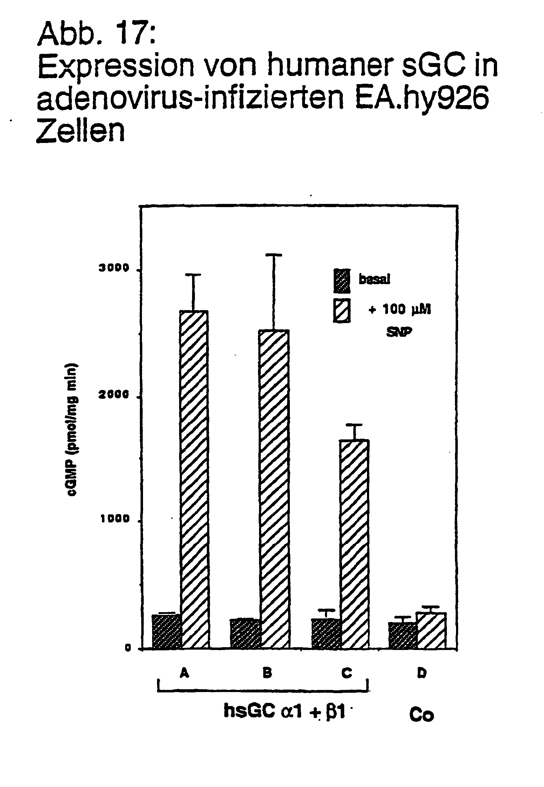

Abbildung 17 zeigt die Stimulierbarkeit der sGC-Aktivität durch 100 µM SNP (Natriumnitroprussid) in EA.hy926-Zellen, die mit den beiden hsGC-Adenoviren Ad5CMVhsGCα1 und Ad5CMVhsGCβ1 koinfiziert worden waren (Proben A - C) und bei uninfizierten EA.hy926-Zellen (Ansatz D). Auf der Y-Achse ist die Bildung von pmol cGMP pro mg Protein und pro Minute aufgetragen. Die dunklen Balken zeigen die basale cGMP-Bildung ohne SNP-Stimulierung, die hellen Balken stellen die cGMP-Bildung nach SNP-Stimulierung dar.

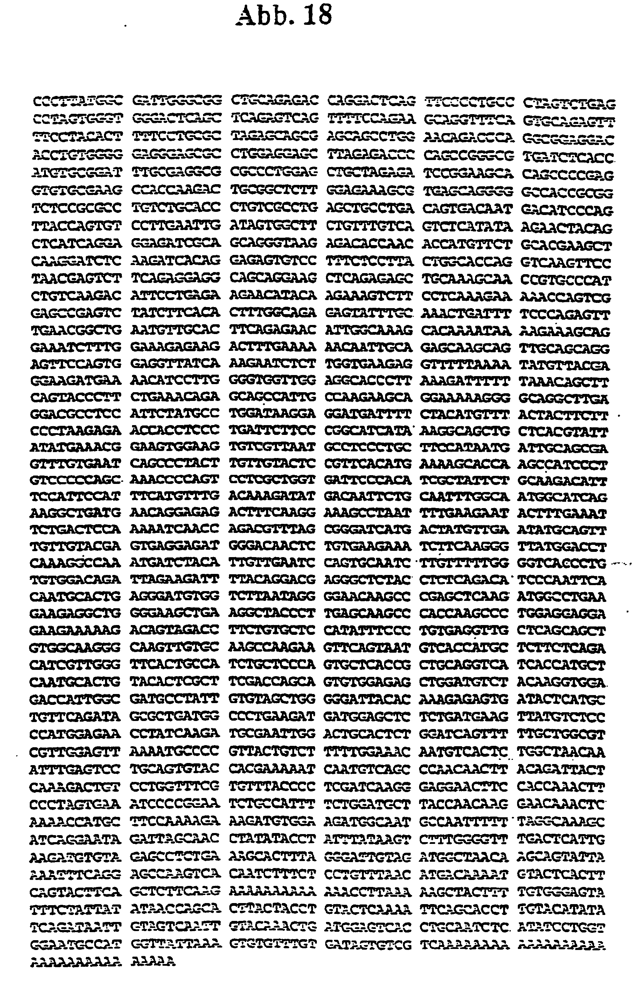

Abbildung 18 zeigt die DNA-Sequenz der humanen löslichen Guanylylcyclase α1 (hsGCα1); SEQ ID NO: 1.

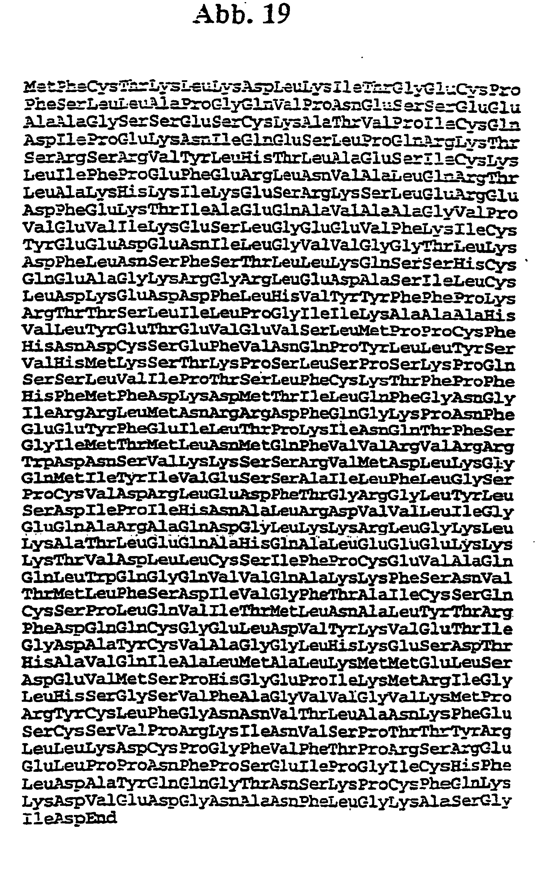

Abbildung 19 zeigt die Aminosäuresequenz der humanen löslichen Guanylylcyclase α1 (hsGCα1); SEQ ID NO: 2.

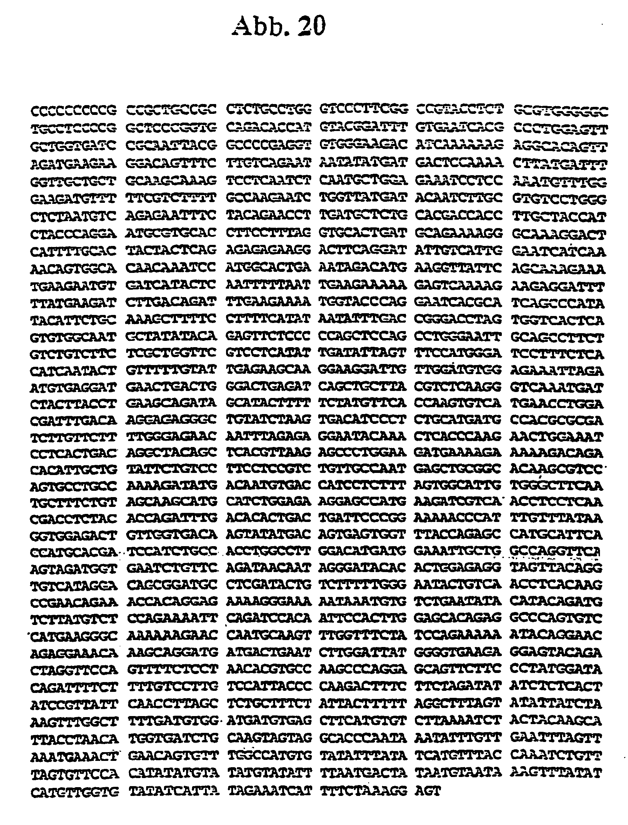

Abbildung 20 zeigt die DNA-Sequenz der humanen löslichen Guanylylcyclase β1 (hsGCβ1); SEQ ID NO: 3.

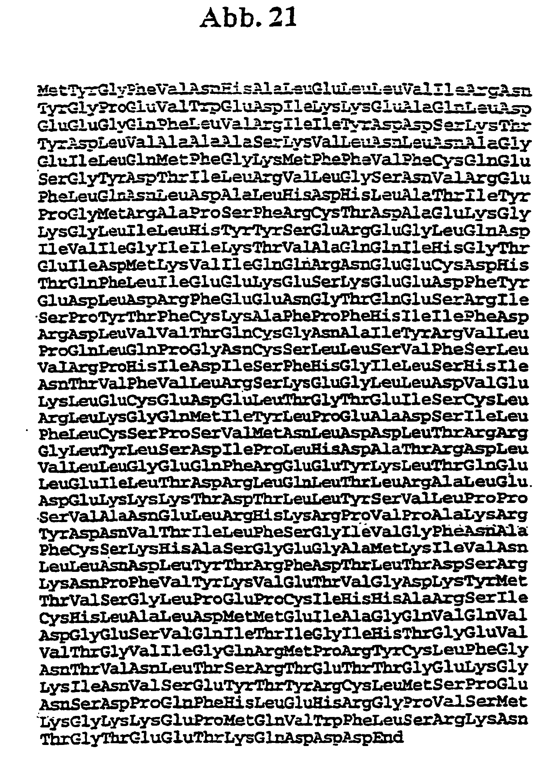

Abbildung 21 zeigt die Aminosäuresequenz der humanen löslichen Guanylylcyclase β1 (hsGCβ1); SEQ ID NO: 4.

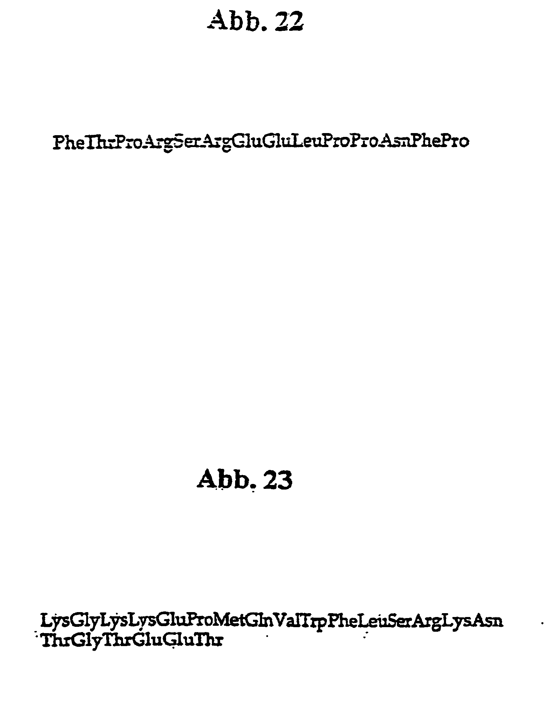

Abbildung 22 zeigt die Aminosäuresequenz des Peptids, das zur Herstellung von Antikörpern gegen die humane lösliche Guanylylcyclase α1 (hsGCα1) verwendet wurde (entspricht Aminosäuren 634-647 der hsGCα1); SEQ ID NO: 5.
Abbildung 23 zeigt die Aminosäuresequenz des Peptides, das zur Herstellung von Antikörpern gegen die humane lösliche Guanylylcyclase β1 (hsGCβ1) verwendet wurde (entspricht Aminosäuren 593-614 der hsGCβ1); SEQ ID NO: 6.

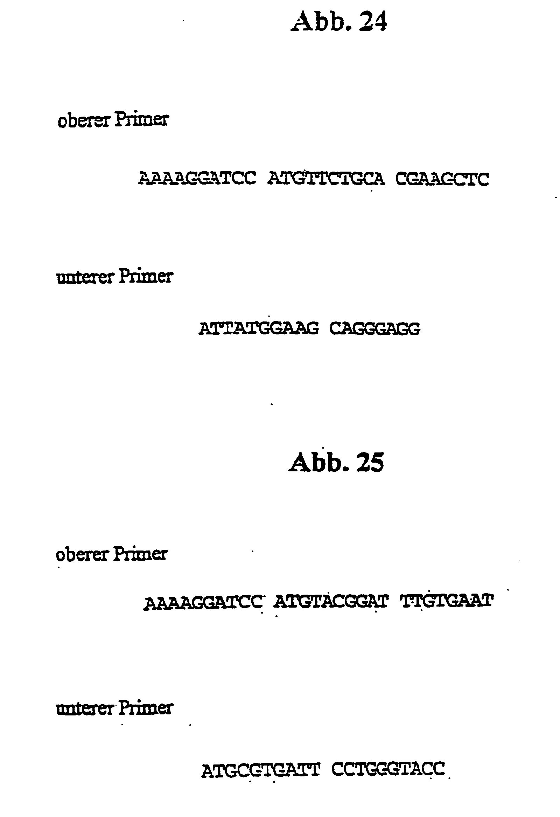

Abbildung 24 zeigt die DNA-Sequenz des PCR-Primerpaares für die humane lösliche Guanylylcyclase a1 (hsGCa1). Oberer Primer (entspricht Nukleotide 524-541 der hsGCα1-cDNA-Sequenz mit angehängter Restriktionsschnittstelle für BamHI); SEQ ID NO: 7. Unterer Primer (entspricht 1249-1232 der hsGCα1-cDNA-Sequenz [Gegenstrang]); SEQ ID NO: 8.
Abbildung 25 zeigt die DNA-Sequenz des PCR-Primerpaares für die humane lösliche Guanylylcyclase β1 (hsGCβ1). Oberer Primer (entspricht Nukleotide 89-106 der hsGCβ1-cDNA-Sequenz mit angehängter Restriktionsschnittstelle für BamHI); SEQ ID NO: 9. Unterer Primer (entspricht Nukleotide 692-711 der hsGCβ1-cDNA-Sequenz [Gegenstrang]); SEQ ID NO: 10.

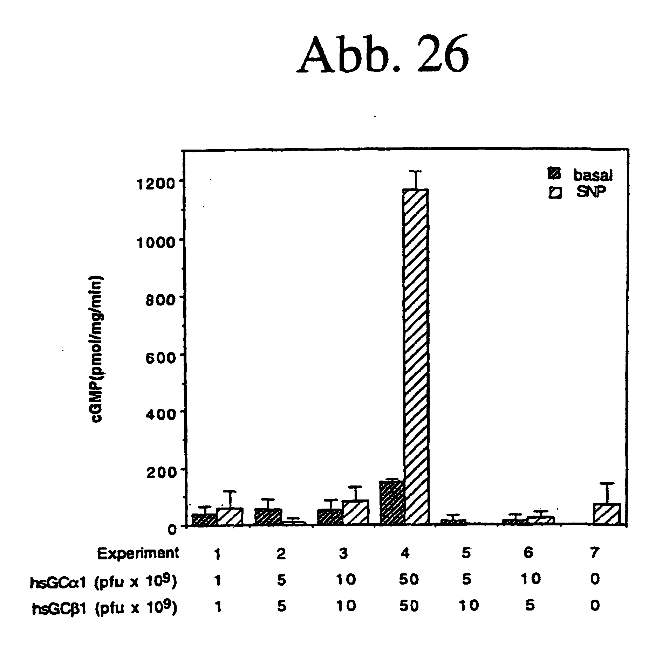

Abbildung 26 zeigt die Stimulierbarkeit der sGC-Aktivität durch 100 µM SNP (Natriumnitroprussid) in ECV 304-Zellen, die mit unterschiedlichen Mengen beider hsGC-Adenoviren Ad5CMVhsGCα1und Ad5CMVhsGCβ1 koinfiziert worden waren (Proben 1-6) und bei uninfizierten ECV 304-Zellen (Probe 7). Auf der Y-Achse ist die Bildung von pmol cGMP pro mg Protein und pro Minute aufgetragen. Die dunklen Balken zeigen die basale cGMP-Bildung ohne SNP-Stimulierung, die hellen Balken stellen die cGMP-Bildung nach SNP-Stimulierung dar.

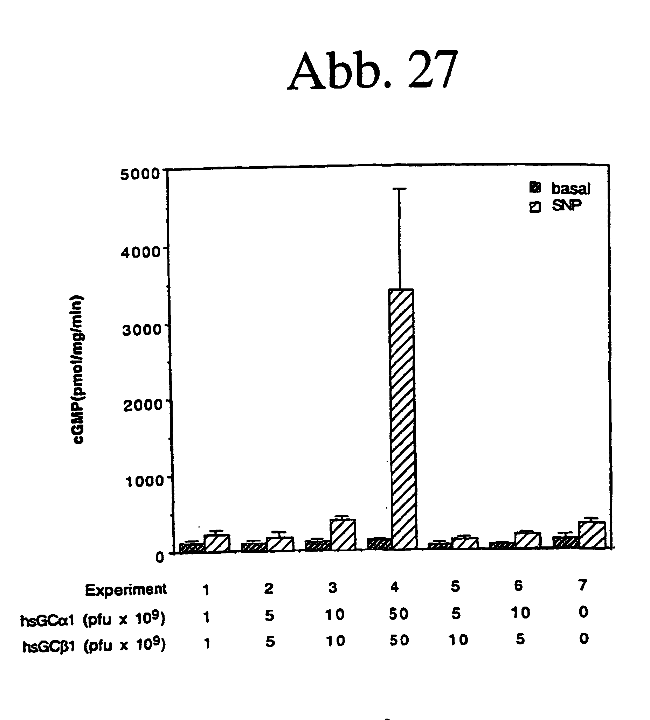

Abbildung 27 zeigt die Stimulierbarkeit der sGC-Aktivität durch 100 µM SNP (Natriumnitroprussid) in A10-Zellen, die mit unterschiedlichen Mengen beider hsGC-Adenoviren Ad5CMVhsGCα1und Ad5CMVhsGCβ1 koinfiziert worden waren (Proben 1-6) und bei uninfizierten A10-Zellen (Probe 7). Auf der Y-Achse ist die Bildung von pmol cGMP pro mg Protein und pro Minute aufgetragen. Die dunklen Balken zeigen die basale cGMP-Bildung ohne SNP-Stimulierung, die hellen Balken stellen die cGMP-Bildung nach SNP-Stimulierung dar.

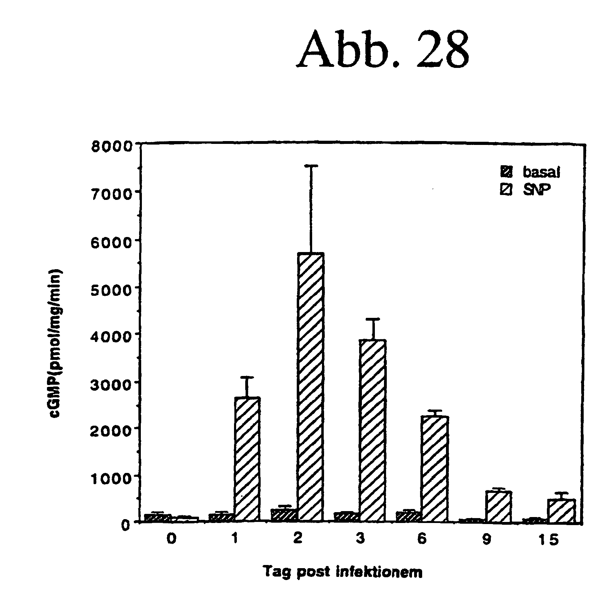

Abbildung 28 zeigt die Stimulierbarkeit der sGC-Aktivität durch 100 µM SNP (Natriumnitroprussid) in ESV 304-Zellen, die mit jeweils 5 x 10¹⁰ hsGC-Adenoviren Ad5CMVhsGCα1und Ad5CMVhsGCβ1 koinfiziert worden waren und danach zu unterschiedlichen Zeitpunkten geerntet wurden und bei uninfizierten ECV 304-Zellen. Auf der X-Achse ist der Erntezeitpunkt der Zellen in Tagen nach Infektion aufgetragen (0, keine Infektion). Auf der Y-Achse ist die Bildung von pmol cGMP pro mg Protein und pro Minute aufgetragen. Die dunklen Balken zeigen die basale cGMP-Bildung ohne SNP-Stimulierung, die hellen Balken stellen die cGMP-Bildung nach SNP-Stimulierung dar.

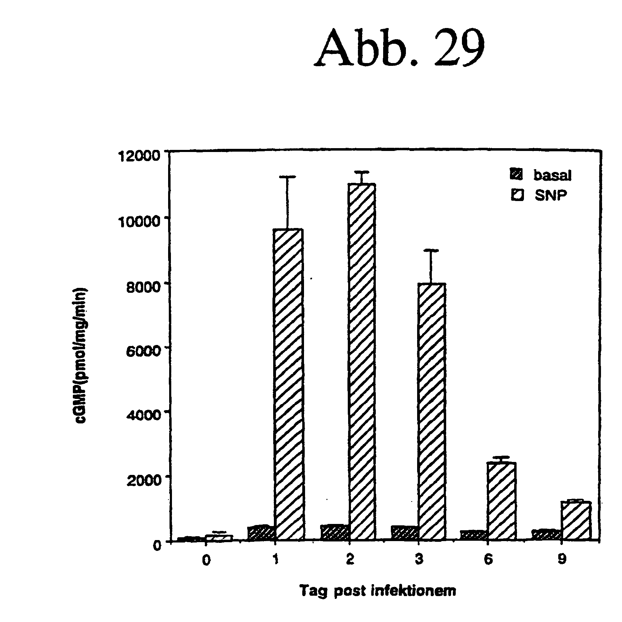

Abbildung 29 zeigt die Stimulierbarkeit der sGC-Aktivität durch 100 pM SNP (Natriumnitroprussid) in A10-Zellen, die mit jeweils 5 x 10¹⁰ hsGC-Adenoviren Ad5CMVhsGCα1und Ad5CMVhsGCβ1 koinfiziert worden waren und danach zu unterschiedlichen Zeitpunkten geerntet wurden und bei uninfizierten A10-Zellen. Auf der X-Achse ist der Emtezeitpunkt der Zellen in Tagen nach Infektion aufgetragen (0, keine Infektion). Auf der Y-Achse ist die Bildung von pmol cGMP pro mg Protein und pro Minute aufgetragen. Die dunklen Balken zeigen die basale cGMP-Bildung ohne SNP-Stimulierung, die hellen Balken stellen die cGMP-Bildung nach SNP-Stimulierung dar.

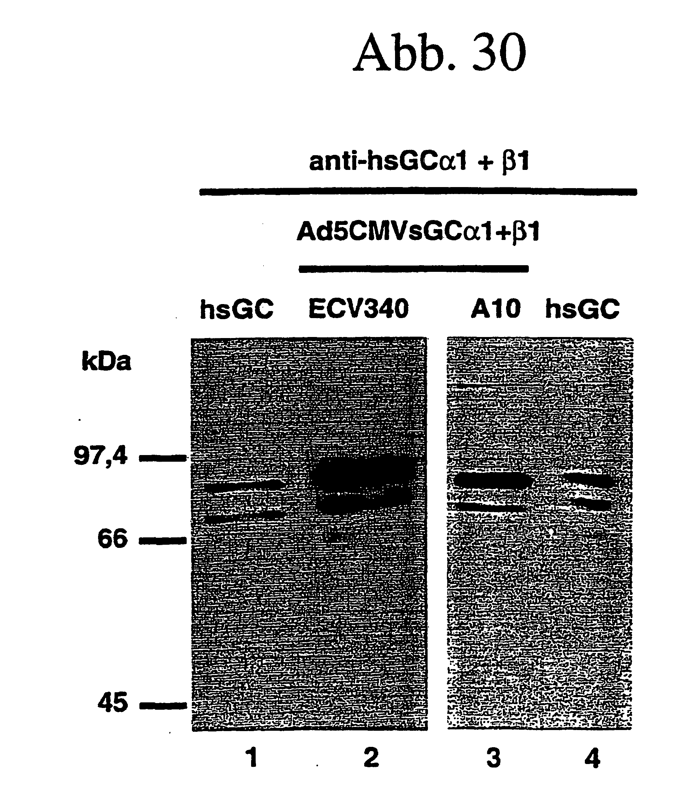

Abbildung 30 zeigt im Proteinimmoblot die Detektion von sGC-Untereinheiten in E304- bzw. A10-Zellen, die mit jeweils 5 x 10¹⁰ hsGC-Adenoviren Ad5CMVhsGCα1und Ad5CMVhsGCβ1 für 2 Tage koinfiziert worden waren, mit den oben beschriebenen Peptidantikörpern gegen hsGCα1und hsGCβ1. Spur 1, gereinigte hsGC (gemäß Abbildung 12); Spur 2, ECV 304-Zellen mit hsGC-Gentransfer; Spur 4, A10 Zellen mit hsGC-Gentransfer; Spur 4, gereinigte hsGC (gemäß Abbildung 12).

### Vorteile der Erfindung und die Lösung der oben angeführten technischen Probleme durch diese Erfindung

1.) Die in der Literatur als sGCα3 und sGCβ3 beschriebenen cDNA-Klone wurden als humane Homologe der sGCα1 und sGCβ1 aus Rind und Ratte identifiziert, und werden nachfolgend als humane sGC α1 (hsGCα1) und humane sGC β1 (hsGCβ1) bezeichnet. Diese sGCα1/β1-Isoform ist nach dem aktuellen Wissensstand wegen ihrer Funktion im kardiovaskulären System als die pharmakologisch wichtigere anzusehen. Da der Originalklon von hsGCα3 untersucht wurde, konnte gezeigt werden, daß nicht hsGCα1 und hsGCα3 parallel existieren, sondern nur die Form hsGCα1. Damit ist ein eindeutiges Zielprotein für ein pharmakologisches Massenscreening und die Gentherapie identifiziert
2.) Durch das erfindungsgemäße Verfahren wird erstmals die funktionell aktive Expression humaner sGC erreicht. Damit ist erstmals das zugehörige Protein durch gentechnische Methoden herstellbar.
3.) Mit den erfindungsgemäßen gegen das Protein sGC gerichteten Peptidantikörpern ist ein Nachweis der Expression in humanen Geweben, sowie die Diagnostik von Dysfunktionen (zu hohe/niedrige oder fehlende sGC-Expression) möglich. Außerdem stellt die Erfindung die technischen Voraussetzungen bereit, die zur weiteren Aufklärung der Transkriptions- und Translationskontrolle von hsGC nötig sind. Die erfindungsgemäßen Peptidantikörper haben den Vorteil, daß sie monospezifisch sind, gegen die humanen Sequenzen gerichtet sind und außerdem ihre Eignung für den Immunoblot mit humanen Geweben nachgewiesen worden ist. Andere Peptidantikörper zeigen diese Eigenschaften nur zum Teil: Harteneck et al. und Guthmann et al. verwenden eine Peptidsequenz (VYKVETVGDKYMTVSGLP), die in Guanylylcyclasen recht hohe Konservierung zeigen, so daß eine Kreuzreaktion auch mit partikulären Guanylylcyclasen (z.B. GC-C) erwartet werden kann. Guthmann et al. verwendet außerdem eine zu hsGCβ1 identische Peptidsequenz (YGPEVWEDIKKEA) und eine bis auf zwei Aminosäureaustausche identische Peptidsequenz zu hsGCα1 (KKDVEEANANFLGKASGID). Allerdings ist die Funktion dieser Antikörper im Immunoblot nur für *angereinigte* hsGC aus humanen Thrombozyten gezeigt worden. Zusätzlich erkannten diese Antikörper gegen hsGCα1 noch ein zweites, unspezifisches Produkt Humbert et al. und Koesling et al. verwendeten eine Peptidsequenz (SRKNTGTEETEQDEN) aus Rinder-sGCβ1, die teilweise (Aminosäuren 1-10) mit dem hier verwendeten Peptid (Aminosäuren 13-22) für hsGCβ1 identisch ist, dessen C-Terminus (Aminosäuren 11-15) jedoch deutlich verschieden von der humanen Sequenz ist. Das Antiserum gegen dieses Peptid wurde jedoch nicht an humanem Protein getestet, sondern nur für die Immunpräzipitation der bovinen sGC verwendet
   Zusätzlich zu den in Abbildung 22 und 23 dargestellten Peptiden und deren immunogenen Fragmenten zur Herstellung der erfindungsgemäßen Antikörpern gegen hsGCα1 oder hsGCβ1 in Kanninchen ist auch die Herstellung von monoklonalen oder polyklonalen Antikörpern gegen das ganze hsGCα1/β1-Protein oder seine Spaltprodukte möglich. Dabei können verschiedene Tierarten (vorzugsweise Maus, Ratte, Kaninchen) zur Antikörperherstellung eingesetzt werden.
4.) Durch die erfindungsgemäße Expression im eukaryontischen Baculovirus/Sf9-System kann die humane l^{∧}sliche Guanylylcyclase α1/β1 in großen Mengen hergestellt werden. Das Anhängen einer Nukleotidsequenz, die für ein für die Affinitätschromatographie geeignetes Polypeptid kodiert (Affinitätstag, z.B. Glutathion-S-Transferase = GST-Tag), mit einer anschließenden Proteaseschnittstelle am N-Terminus der cDNA der **α**1-Untereinheit erlaubt die schnelle und einfache Aufreinigung des koexprimierten, dimeren Proteins durch einen einzigen affinitätschromatogranschen Schritt Das anhängende Affinitätstag wird anschließend durch Proteaseverdau wieder entfernt, wodurch hinsichtlich der Primärstruktur ein dem nativen identisches Protein erhalten wird. Durch diese revolutionär schnelle und saubere Gewinnung großer hochreiner Mengen an funktioneller humaner sGC ergeben sich neue Möglichkeiten für ein Massenscreening nach spezifischen Aktivatoren und Inhibitoren sowie für die pharmakologische Charakterisierung der potentiellen Arzneistoffe.
   Eine Aufreinigung von hsGCα1/β1 kann auch erfolgen durch IonenaustauschChromatographie, Gelfiltration, Immunaffinitäts-Chromatographie und andere chromatographische Verfahren, z.B. an ATP-, GTP-, cGMP- oder Blue-Sepharose, und anderen derartigen Chromatographiemedien.
5.) Das erfindungsgemäße Verfahren ist in identischer Weise für andere Isoformen des Enzyms bei Mensch, Ratte und Rind einsetzbar. In dem erfindungsgemäßen Verfahren können verschiedene Affinitätstagstags (z.B. Histidin-Oligomer) und verschiedene Expressionssysteme wie z.B. E. coli eingesetzt werden. Andere Bereiche aus der hsGCα1- und hsGCβ1-Sequenz können ebenfalls zur Herstellung von Antikörpern gegen Peptide oder das ganze Protein verwendet werden.
6.) Für die moderne Pharmakaentwicklung ist die Verfügbarkeit großer Mengen eines isolierten humanen Proteins in hoher Reinheit und von hoher Qualität essentiell. Diese Voraussetzung war für die Suche nach Alternativen zu den klassischen NO-Donoren bisher nicht gegeben. Ein Massenscreening nach spezifischen Aktivatoren oder Inhibitoren ist im Tierversuch zu teuer und wegen Speziesunterschieden, möglichen Nebenwirkungen und Effekten auf andere Isoformen nicht sinnvoll. Zellkultursysteme haben den Nachteil, daß nur mit erheblichem Mehraufwand festgestellt werden kann, wo Substanzen innerhalb der Signalkaskaden ihre Wirkungen entfalten. Außerdem ist die Zellkultur kostenintensiv und empfindlich. Die Aufreinigung eines Proteins aus tierischen Geweben ist, im Vergleich zur Expression eines Proteins mit rekombinanter DNA-Technologie, aufwendiger bei geringerer Ausbeute. Vor allem aber sind die Ergebnisse eines Pharmakascreenings aufgrund der Speziesunterschiede nur bedingt übertragbar. Die rekombinante hsGCα1/β1 dagegen ist durch die erfindungsgemäßen Verfahren in großen Mengen kostengünstig verfügbar und liefert im Screening eindeutige Aussagen über Modulation, Aktivierung oder Inhibition dieses speziellen humanen Enzyms. Speziesunterschiede und mangelnde Übertragbarkeit auf den Menschen sind durch Verwendung eines humanen Enzyms a priori ausgeschlossen.
   Weiterhin steht so für Kristallisation und Strukturaufklärung hsGC in großen Mengen und ausreichender Reinheit zur Verfügung. Hierdurch ist eine wichtige Voraussetzung für ein rationelles Pharmako-Design mittels Molecular Modelling erfüllt.
7.) Zusätzlich zur Verwendung der isolierten hsGCα1/β1 können intakte Sf9-Zellen, die durch Infektion mit den hier beschriebenen rekombinanten Baculoviren hsGCα1/β1 exprimieren, für in-vitro-Versuche eingesetzt werden.
8.) Durch Gentransfer läßt sich eine vorübergehende (z.B. mit adenoviralen Vektoren) oder dauerhafte Überexpression erreichen, wodurch der cGMP-Spiegel auch bei geringer NO-Konzentration oder schlechterer Aktivierung der sGC durch Peroxynitrit-Bildung erhöht werden kann. Dieser Ansatz bietet außerdem konzeptionelle Vorteile gegenüber einem Gentransfer von NOS (NO-Synthase), da mit dem erfindungsgemäßen Ansatz die Bildung von zytotoxischem, und bezüglich der Aktivierung von sGC weniger effektivem Peroxynitrit umgangen wird. Außerdem ließe sich (z.B. für therapeutische Zwecke ) durch Gentransfer von sGC-Mutanten mit erhöhter Basalaktivität unabhängig von NOS, NO, NO-Donoren oder sGC-Modulatoren eine dauerhafte Erhöhung des cGMP-Spiegels erreichen.
9.) Zusätzlich zu dem beschriebenen Reinigungsverfahren für hsGCα1/β1 ist eine Aufreinigung aus Sf9-Zellen nach Infektion mit den beschriebenen Baculoviren mit hsGCα1- oder hsGCβ1-cDNA auch möglich, wenn mit hsGCα1-Baculoviren ohne GST-Tag (GST-Tag = angehängte Glutathion-S-Transferase-Sequenz aus *Schistosoma japonicum*) und hsGCβ1-Baculoviren mit GST-Tag koinfiziert wird, sowie bei Koinfektion von hsGCα1-Baculoviren mit GST-Tag und hsGCβ1-Baculoviren mit GST-Tag.
   Außerdem ist zusätzlich zum Einsatz von loser Glutathion-Sepharose 4B auch die Verwendung einer Säule mit Glutathion-Sepharose 4B möglich.
   Eine Aufreinigung kann auch erfolgen, indem *nach* einer Elution des dimeren Fusionsproteins GST-hsGCα1/β1 mittels reduziertem Glutathion von der Glutathion-Sepharose 4B dieses Fusionsprotein mit Thrombin verdaut wird. Nach Dialyse (zur Entfernung des reduzierten Glutathions) kann dann das abgespaltene GST-Tag durch erneute Affinitätschromatographie an Glutathion-Sepharose 4B aus dem Gemisch entfernt werden.
10.) Das erfindungsgemäße Verfahren kann auch für die humane lösliche Guanylylcyclase α2 (GenBank: x63282) und ein eventuell existierendes humanes Homolog zur löslichen Guanylylcyclase β2 (aus Ratte; GenBank: m57507), sowie für andere humane lösliche Guanylylcyclasen angewendet werden (in allen hier aufgeführten technischen Varianten).
11.) Durch den adenoviralen, somatischen Gentransfer von hsGCα1 bzw. hsGCβ1 mittels Koinfektion durch Ad5CMVhsGCα1 bzw. Ad5CMVhsGCβ1 konnte in Endothel (ECV 304)- und glatten Muskelzellen (A10) gezeigt werden, dass die intrazelluläre sGC-Aktivität, gemessen als cGMP-Gehalt nach Stimulation von sGC mit SNP, von nicht detektierbaren Spiegeln (≤ 100 pmol/mg/min) auf 12-fach höhere Werte stimuliert werden kann. Es zeigte sich ein Konzentrationsoptimum hinsichtlich der Menge an Adenovirus (pfu) sowie, entsprechend der homodimeren Struktur des Enzyms, eines äquimolaren Verhältnis (bezogen auf pfu) von beiden Adenoviren. Dieser Effekt auf intrazellulär nachweisbare sGC-Aktivität ließ sich 15 Tage nach 1-maliger Infektion noch nachweisen, ein für die beanspruchten Indikationen ausreichender Zeitraum.
   Die Beispiele erläutern die Erfindung.

### Beispiel 1

### Die korrigierte Sequenz von hsGCα1 und hsGCβ1

Die Orginalklone der humanen Isoformen sGCα3 und sGCβ3 (Giuili et al., 1992) wurden erneut sequenziert. Während die Sequenz des sGCβ3-Klones bestätigt wurde (siehe SEQ ID NO: 3 und Abb. 20), ergab die Sequenzierung von sGCα3, daß die Originalpublikation (Giuili et al., 1992) 19 Sequenzierfehler enthält, die in einer Übersicht in Abb. 3 zusammengefaßt sind. Die entsprechend korrigierte α3-cDNA-Sequenz ist in SEQ ID NO: 1 und Abb. 18 dargestellt. Die abgeleitete Aminosäuresequenz ist in SEQ ID NO: 2 und Abb. 19 dargestellt. Die korrigierte Sequenz (siehe SEQ ID NO: 1 und Abb. 18) ist darüberhinaus identisch mit der inder GenBank veröffentlichten humanen sGCα1-Sequenz (Accesion-Nr. U58855), wobei allerdings der 5'-untranslatierte Bereich der hier vorgestellten Sequenz um 506 Basenpaare länger ist "sGCα3" wird daher jetzt als humane sGCα1 (hsGCα1) klassifiziert. Damit ist gezeigt, daß beim Menschen nicht zwei verschiedene hsGCα-Untereinheiten α1 und α3 vorkommen, die für die kardiovaskuläre Forschung von Bedeutung sein könnten, sondern - analog zur Situation bei Rind und Ratte - nur hsGCα1.

**Tabelle 1**

| Revidierte Terminologie von löslichen Guanylylcyclase-cDNAs und -proteinen, und ihr Nachweis in humanem Gewebe. | | |
|---|---|---|
| | humane sGC-Untereinheiten | |
| | α | β |
| Isoform 1 | cDNA und Protein nachweisbar | cDNA und Protein nachweisbar |
| | aktiv, wenn koexprimiert | |
| Isoform 2 | cDNA nachweisbar | |
| | aktiv, wenn mit boviner β1 koexprimiert | |

Die Expression von sGCα1- und sGCβ1-mRNA in humanen Geweben wurde mittels PCR gezeigt (Abb. 4). Die Amplifikation eines hsGCβ1-Fragmentes mit einem PCR-Primerpaar (5'-AAAAGGATCCATGTACGGATTTGTGAAT-3' = Nukleotide 89-106 der hsGCβ1-cDNA-Sequenz mit angehängter Restriktionsschnittstelle; 5'-ATGCGTGATTCCTGGGTACC-3' = 692-711 der hsGCβ1-cDNA-Sequenz) bei einer Annealing-Temperatur von 54°C ergab jeweils eine spezifische Bande in cDNA-Banken aus Hirn, Herz, Niere, Lunge, Pankreas und Skelettmuskel. Die Identität des amplifizierten Fragmentes wurde durch Sequenzierung bestätigt. Die Amplifikation eines hsGCα1-Fragmentes mit einem PCR-Primerpaar (5'-AAAAGGATCCATGTTCTGCACGAAGCTC-3' = Nukleotide 524-541 der hsGCα1-cDNA-Sequenz mit angehängter Restriktionsschnittstelle; 5'-ATTATGGAAGCAGGGAGG-3' = 1249-1232 der hsGCα1-cDNA-Sequenz) bei einer Annealing-Temperatur von 54° C ergab jeweils eine spezifische Bande in cDNA-Banken aus Herz (Abb. 4A) und Lunge (nicht gezeigt). Die Sequenzierung der Fragmente ergab in jedem Fall die korrigierte hsGCα1-Sequenz; die von Giuili et al. publizierte "sGCα3"-Sequenz wurde nicht gefunden. Damit wurde gezeigt, daß beim Menschen nur eine hsGCα1/β1 vorhanden ist, während die mutmaßliche hsGCα3/β3 lediglich auf Sequenzierfehlern beruht Dadurch ergibt sich für die kardiovaskuläre Forschung ein klares Bild bezüglich der sGC-Isoform, die im Targetprotein für das Pharmaka-Screening ist

### Beispiel 2

### Konstruktion von rekombinanten Baculoviren für die Expression humaner sGCα und sGCβ in Insektenzellen

Zum Nachweis, daß hsGCα1 und hsGCβ1 funktionelles, heterodimeres sGC-Protein konstituieren können, wurden die beiden cDNAs in Baculovirus eingebracht, mit dessen Hilfe ein rekombinantes Protein unter der Kontrolle des starken Polyhedrinpromotors in Insektenzellen (Sf9-Zellen) exprimiert wurde. Zur Herstellung der rekombinanten Baculoviren wurde der Baculovirus-Transfervektor pVL1393 (Fa. Pharmingen, San Diego, California, USA; Abb. 5) bzw. der Baculovirus-Transfervektor pAcG2T (mit Glutathion-S-Transferase-Sequenz aus *Schistosoma japonicum* und Thrombinschnittstelle; Fa. Pharmingen; Abb. 6) verwendet, in den die Fremdgene (hsGCα1 und hsGCβ1) kloniert wurden. Die Kotransfektion eines solchen rekombinanten pVL1393- oder pAcG2T-Plasmids mit BaculoGold Baculovirus-DNA (Pharmingen) erlaubte die direkte Isolation der durch homologe Rekombination entstandenen gentechnisch veränderten Baculoviren mit hsGCα1- oder hsGCβ1-cDNA aus dem Zellkulturmedium.

Die Konstruktion von pVL1393-hsGCβ1 ist in Abb. 7 schematisch dargestellt (identische Vorgehensweise für pAcG2T-hsGCβ1). Es wurde der kodierende Bereich der hsGCβ1 cDNA mit der 3'-untranslatierben Region, jedoch ohne die 5'untranslatierte Region, in pVL1393 kloniert. Dazu wurde mittels PCR mit den Primern A und B eine BamHI-Schnittstelle unmittelbar in 5'- Richtung des für das Start-Methionin codierenden Codons eingeführt. Das so amplifizierte Fragment 1 wurde mit BamHI/KpnI verdaut; Fragment 2 wurde mit KpnI/EcoRI aus dem sGCβ1-cDNA-Klon isoliert. Die Fragmente 1 und 2 sowie der BamHI/EcoRIgeöffnete Vektor wurden ligiert (siehe Abb. 7).

Die Konstruktion von pVL1393-hsGCα1 ist in Abb. 8 schematisch dargestellt (identische Vorgehensweise für pAcG2T-hsGCα1). Es wurde der kodierende Bereich der hsGCα1 cDNA mit der 3'-untranslatierten Region, jedoch ohne die 5'untranslatierte Region, in pVL1393 kloniert. Dazu wurde mittels PCR mit den Primern C und D eine BamHI-Schnittstelle unmittelbar in 5'-Richtung des für das Start-Methionin codierenden Codons eingeführt. Das so amplifizierte Fragment 3 wurde mit BamHI/BsaAI verdaut; Fragment 4 wurde mit BsaAI/EcoRI aus dem sGCα1-cDNA-Klon isoliert. Die Fragmente 3 und 4 sowie der BamHI/EcoRIgeöffnete Vektor wurden ligiert (siehe Abb. 8).

Zur Herstellung der rekombinanten hsGCα1- und hsGCβ1-Baculoviren wurde jeweils der Baculovirus-Transfervektor (pVL1393-hsGCα1, pAcG2T-hsGCα1, pVL1393-hsGCβ1, pAcG2T-hsGCβ1) mit Baculovirus-DNA (Baculo-Gold; Fa. Pharmingen, San Diego, California, USA) in Sf9-Monolayer-Zellkulturen kotransfiziert. Die Zellen wurden dazu bei 27°C in IPL-41-Medium (Gibco) kultiviert, das mit 10 % (Vol/Vol) fötalem Kälberserum (Biochrom), 4 % (Vol/Vol) Tryptose-Phosphat-Broth (Gibco), 1 % (Vol/Vol) Pluronic F68 (Gibco), 0,5 % Amphotericin B (Gibco), 80 µg/ml Gentamycinsulfat (Gibco) und 0.5 mM δ-Aminolävulinsäure (Merck) suppelementiert war. Rekombinante hsGCα1- und hsGCβ1-Baculovirenklone wurden mittels Plaque-Reinigung aus dem Kulturmedium gewonnen. Zur Herstellung von Virusstammlösungen mit hohem Titer wurden Sf9-Schüttelkulturen (0,5 x 10⁶ Zellen/ml) mit einer M.O.I. (multiplicity of infection) von 0,1 pfu/Zelle (pfu = Plaque-bildende Einheiten) infiziert und 6 Tage nach der Infektion geerntet.

### Beispiel 3

### Produktion von rekombinanter hsGcα1 und hsGCβ1 in Sf9-Zellen

Jeweils 10 rekombinante hsGCα1- und hsGCβ1-Baculovirenklone wurden bezüglich der Expression von rekombinantem Protein in Sf9-Zellen getestet Dazu wurden Sf9-Monolayer-Zellkulturen mit plaquegereinigtem, rekombinatem hsGCα1- oder hsGCβ1-Baculovirus infiziert, 5 Tage bei 27°C kultiviert, mit einem Gummischaber geerntet, in 0,5 ml Lysispuffer (25 mM TEA, pH 7.8, 50 mM NaCl, 1mM EDTA, 5 mM DTT, 1 µM Leupeptin, 0,5 mg/l Trypsininhibitor) resuspendiert und mit Ultraschall homogenisiert ("Sonifier 250", "Standard"-Spitze, Branson; 15-mal, "duty cycle": 15 %, Intensität: 1). Nach Zentrifugation der Homogenate bei 20.000 x g wurden Überstand und Pellet in einer SDS-PAGE analysiert. Drei der hsGCβ1-Baculovirenklone und zwei der hsGCα1-Baculovirenklone lieferten rekombinantes Protein in Mengen, die in der unlöslichen Fraktion durch Färbung mit Coomassie-Brilliant-Blue R250 sichtbar gemacht werden konnten. Rekombinante humane sGCα und sGCβ (rhsGCα und rhsGCβ) wanderten mit einem apparenten Molekulargewicht von 79,5 (hsGCα1) bzw. 68,5 kDa (hsGCβ1), was den aus der abgeleiteten Aminosäuresequenz vorhergesagten Molekulargewichten sehr nahe kommt (77,5 bzw. 70,5 kDa) (repräsentative Klone in Abb. 9). Die Baculovirenklone, die im Immunoblot die höchsten Expression von rekombinantem Protein zeigten, wurden für die Expression funktioneller heterodimerer hsGC verwendet [siehe Beispiele 4 bis 7.]

### Beispiel 4

### Rekombinante Humane sGC in intakten Insektenzellen ist aktiv und NO-stimulierbar

Zur Herstellung funktioneller heterodimerer humaner sGC wurden rhsGCα1 und rhsGCβ1 über die rekombinaten Baculoviren in Sf9-Zellen koexprimiert. Dazu wurden Sf9-Monolayerkulturen [2,5 x 10⁶ Zellen/Petrischale, ø 90 mm; Supplemente siehe Ziffer 2)] mit einer M.O.I. (multiplicity of infection) von 2 pfu/Zelle für jeden rekombinanten Baculovirus (hsGCα1 und hsGCβ1; beide ohne GST-Tag) koinfiziert und für 48 h bei 27°C kultiviert Die basale sowie die NO-stimulierbare Aktivität der sGC in den Zellen wurde durch Messung des cGMP-Gehaltes der Zellen in Anwesenheit des Phosphodiesterase-Inhibitors IBMX (3-Isobutyl-1-methylxanthin) bestimmt.

Zur Bestimmung des cGMP-Gehaltes wurde das Kulturmedium durch Krebs-Ringer-Puffer (KRB), welcher zusätzlich 1mM IBMX enthielt, ersetzt (119 mM NaCl; 4,74 mM KCl; 2,54 mM CaCl₂; 1,19 mM MgSO₄; 1,19 mM KH₂PO₄; 25 mM NaHCO₃; 10 mM HEPES, pH 7,4; 0,1% BSA), und die Zellen für eine Stunde bei 27°C kultiviert Anschließend wurden die Zellen mit eiskaltem KRB gewaschen und mit einem Gummischaber in 1 ml eiskaltem Ethanol (80 %) geerntet. Die Zellen wurden mit Ultraschall homogenisiert [siehe oben unter Ziffer 3.)] und für 20 min bei 20000 × g zentrifugiert. Der Überstand wurde in einem Vakuumkonzentrator getrocknet und der Rückstand anschließend in 25 mM TEA, pH 7,8 resuspendiert. Der cGMP-Gehalt wurde im RIA bestimmt (Biotrend).

Die Koexpression von rhsGCα1 und rhsGCβ1 führte zur Bildung funktioneller sGC mit basaler Aktivität in Sf9-Zellen (Abb. 10): Während nicht infizierte Sf9-Zellen etwa 0,1 pmol cGMP/10⁶ Zellen enthielten (nicht gezeigt), wurde in rhsGC-exprimierenden Zellen etwa 20 pmol cGMP/10⁶ Zellen gefunden (Abb. 10). Diese basale Aktivität rekombinanter hsGC war durch einen NO-Donor, SNP (Natriumnitroprussid), stimulierbar. Wurden die Zellen vor der Ernte für 2 min mit 10, 100 oder 1000 µM SNP inkubiert, stieg der cGMP-Gehalt konzentrationsabhängig auf den bis zu 50-fachen Wert an (Abb. 10).

### Beipiel 5

### Rekombinante humane sGC in Insektenzellextrakten ist aktiv und NO-stimulierbar

Die Aktivität der rekombinanten hsGC (nach Expression mit den oben beschriebenen rekombinanten Baculoviren) wurde nicht nur in intakten Sf9-Zellen nachgewiesen, sondern auch in Sf9-Zellextrakten. Zur Herstellung solcher Extrakte wurden Sf9-Schüttelkulturen [2 x 10⁶ Zellen/ml; Supplemente siehe Beipiel 2] mit einer M.O.I. (multiplicity of infection) von 1 pfu/Zelle für jedes Virus (hsGCα1 und hsGCβ1; beide ohne GST-Tag) koinfiziert und bei 27°C kultiviert. 0, 24, 48, 72, 96 und 118 h nach der Infektion wurden Proben gezogen (4 ml), die Zellen sedimentiert, in 1 ml Lysispuffer resuspendiert und mit Ultraschall homogenisiert [siehe Beispiel 3]. Die Homogenate wurden 15 min bei 20000 x g zentrifugiert, und das unlösliche Pellet wurde erneut in Lysispuffer resuspendiert. Die Proben wurden auf 50 % Glycerin (Vol/Vol) gebracht und bei -20°C gelagert. Die Proteinkonzentrationen wurden spektrophotometrisch mit der Standardmethode von Bradford bestimmt (Bradford, 1976). Die sGC-Aktivität wurde durch die Umsetzung von [α³²P]-GTP zu [³²P]-cGMP bestimmt (Schultz und Böhme, 1984). Die Reaktionsansätze enthielten 50 mM TEA (pH 7,4), 3 mM MgCl₂, 3 mM DTT, 1 mM IBMX, 1 mM cGMP, 5 mM Kreatinphosphat, 0,25 mg/ml Kreatinkinase und 500 µM GTP in einem Gesamtvolumen von 100 µl, und wurden für 10 min bei 37°C inkubiert Die Reaktionen wurden durch gleichzeitige Zugabe von Zellextrakt und den sGC-Aktivatoren SNP, CO oder YC-1 gestartet. Das gebildete [³²P]cGMP wurde gemessen wie beschrieben (Schultz und Böhme, 1984).
Basale rhsGC-Aktivität (d.h. cGMP-Bildung durch rshGC *ohne* Aktivierung des Enzyms durch Zugabe von NO oder anderen Aktivatoren) wurde hauptsächlich in der löslichen Sf9-Zellfraktion gefunden (Abb. 11A), war 72 h nach Infektion der Zellen maximal, und wurde mit 100 µM SNP bis zu 5-fach stimuliert (Abb. 11A). Die Pelletfraktion enthielt zu keinem Zeitpunkt meßbare basale sGC-Aktivität; unter SNP-Stimulation wurde eine geringe sGC-Aktivität gefunden (Abb. 11B).

### Beispiel 6

### Einfluß von YC-1 und ODQ auf rekombinante humane sGC

Mit YC-1 (3-(5'-Hydroxymethyl-2'-furyl)-1-benzylindazol) und ODQ (1H-[1,2,4]oxadiazolo[4,3,-a]chinoxalin-1-on) sind Substanzen beschrieben, welche die Aktivität von sGC spezifisch beeinflussen können. Es wurde daher untersucht, ob dies auch für rhsGC zutrifft.
Nach Expression mit den oben beschriebenen rekombinanten Baculoviren (ohne GST-Tag) wurde die rhsGC in intakten Sf9-Zellen durch YC-1 aktiviert, und auch der NO-potenzierende Effekt wurde gefunden: der cGMP-Gehalt rhsGCexprimierender Zellen wurde durch Inkubation mit 10 µM YC-1 für 2 min um das 3,4-fache erhöht (Abb. 10A). 100 µM YC-1 hatten den gleichen Effekt (Abb. 10A). Wurden die Zellen gleichzeitig mit YC-1 und 100 µM SNP behandelt, verdoppelten sich die cGMP-Spiegel, verglichen mit den durch SNP allein erreichten cGMP-Spiegeln (Abb. 10A). Ähnliche Resultate wurden mit rhsGC in Zellextrakten erzielt ODQ ist als selektiver Inhibitor NO-stimulierter sGC beschrieben, der jedoch nicht die Basalaktivität hemmt (Garthwaite et al., 1995). In rhsGC-exprimierenden Sf9-Zellen (nach Expression mit den oben beschriebenen rekombinanten Baculoviren, ohne GST-Tag) hatte ODQ keinen Einfluß auf die basalen cGMP-Spiegel; die Stimulation von rhsGC in intakten Zellen mit SNP wurde jedoch durch gleichzeitige Inkubation mit ODQ inhibiert (Abb. 10B).

### Beispiel 7

### Gewinnung der gereinigten humanen Guanylylcyclase α1/β1 (hsGCα1/β1)

Zur Reinigung der rekombinanten humanen sGCα1/β1 aus den Sf9-Zellen, wurde ein rekombinanter Baculovirus [siehe Beispiel 2] eingesetzt, bei dem ein Fusionsprotein aus hsGCα1 und anhängendem GST [der sogenannte GST-Tag; GST = Glutathion-S-Transferase aus *Schistosoma japonicum* ; vgl. Bsp. 2] gebildet wird. Über diesen GST-Tag, der mit hoher Affinität Glutathion bindet, ist eine spezifische Affinitätschromatographie an Glutathion-Sepharose 4B, (Fa. Pharmacia, Freiburg) möglich. Die mit hsGCα1-Baculoviren (mit GST-Tag) und hsGCβ1-Baculoviren (ohne GST-Tag) koinfizierten Sf9-Zellen wurden in 25 mM Triethanolamin pH 7,8 / 1 mM EDTA / 5 mM DTT / 1 µM Leupeptin / 0,5 µg/ml Trypsininhibitor / 0,2 mM PMSF lysiert (30 min hypotone Lyse bei 4°C). Nach Zugabe von NaCl auf 75 mM Endkonzentration wurde das Homogenat für 1h bei 75000 x g und 4°C abzentrifugiert und der Überstand mit GSH-Sepharose 4B für eine Stunde bei Raumtemperatur gemischt. Die Glutathion-Sepharose 4B wurde dann bei 500 x g für 5 min abzentrifugiert und der Überstand abgenommen. Die Glutathion-Sepharose 4B wurde mit dem 10fachen Volumen 50 mM Tris-HCl (pH 8,0) / 150 mM NaCl / 2,5 mM CaCl₂ / 0,1 % β-Mercaptoethanol versetzt, für eine Minute gemischt und erneut bei 500 x g für 5 min abzentrifugiert Der Überstand wurde wiederum abgenommen und die Glutathion-Sepharose 4B nochmals auf die gleiche Art gewaschen. Die Elution erfolgte dadurch, daß das hsGCα1-Protein (mit dem daran gebundenen hsGCβ1) durch Thrombin an der spezifischen Schnittstelle von dem GST-Tag getrennt wurde, der an die Glutathion-Sepharose 4B gebundenen blieb. Der Thrombinverdau erfolgte dabei in 50 mM Tris-HCl (pH 8,0) / 150 mM NaCl / 2,5 mM CaCl₂ / 0,1 % β-Mercaptoethanol mit 0,25 bis 1 Einheiten Thrombin / ml Puffer für 1 oder 3 Stunden bei Raumtemperatur. Die Glutathion-Sepharose 4B (mit dem GST-Tag) wurde erneut 5 min bei 500 x g abzentrifugiert und der Überstand mit der abgespaltenen hsGCα1/β1 abgenommen. Eine andere Elutionsmethode erfolgte durch Zugabe von 50 mM Tris-HCl (ph 8,0) / 5 mM reduziertes Glutathion und Mischen für 30 min bei Raumtemperatur. Dadurch wurde hsGCα1/β1 mitsamt dem anhängenden GST-Tag von der Glutathion-Sepharose 4B abgelöst Nach Zentrifugation bei 500 x g für 5 min wurde der Überstand mit darin gelöstem GSThsGCα1/β1 abgenommen.

Durch die Elution mit Thrombin ergibt sich mit einem einzigen, affinitätschromatogranschen Schritt eine doppelte Selektivität:
1.) Es binden nur Proteine, die eine Affinität zu reduziertem Glutathion zeigen.
2.) Von diesen Proteinen werden nur diese (als Spaltprodukte) eluiert, die von Thrombin gespalten werden. Die Abtrennung des Thrombins kann über eine p-Aminobenzamidinsäule erfolgen, an die das Thrombin spezifisch bindet.

Abb. 12 zeigt im Vergleich zum Lysat der infizierten Sf9-Zellen die spezifische Anreicherung der sGC-Aktivität nach Elution von der GSH-Sepharose 4B mit Glutathion.

Abb. 14 zeigt im Immunoblot die Bindung von GST-hsGCα1 an Glutathion-Sepharose 4B und die Abspaltung des hsGCα1 vom GST-Tag durch Thrombin.

Abb. 15 zeigt im mit Coomassie-Brilliant-Blue-R250 gefärbten SDS-Polyacrylamid-Gel den Nachweis der Reinigung von koexprimierter hsGCα1 mit GST-Tag und hsGCβ1 ohne GST-Tag durch Affinitätschromatographie an Glutathion-Sepharose 4B. Bei Elution mit reduziertem Glutathion waren nur zwei Banden zu erkennen, die GST-hsGCα1 (größeres Produkt) und hsGCβ1 (kleineres Produkt) entsprechen (nachgewiesen im Immunoblot). Wurde dagegen mit Thrombin eluiert (0,25 oder 0,5 oder 1 Einheit/ml für 3 Stunden bei Raumtemperatur), so war die untere Bande identisch (hsGCβ1; Molekulargewicht wurde nach Laufverhalten geschätzt auf ca. 70 kDa), die obere Bande war im Vergleich zur Elution mit Glutathion deutlich kleiner (hsGCα1; Molekulargewicht wurde geschätzt auf ca. 80 kDa), da bei der Thrombinelution der GST-Tag abgespalten wird. Dies entspricht in etwa den aufgrund der abgeleiteten Aminosäuresequenz vorhergesagten Molekulargewichten von 77,5 kDa für hsGCα1 und 70,5 kDa für hsGCβ1. Bei der Elution durch Thrombin war anders als bei der Elution durch reduziertes Glutathion noch eine sehr kleine Bande von etwa 25 kDa zu erkennen, die Thrombin selbst darstellen dürfte. Das Thrombin kann aus dem Eluat durch eine p-Aminobenzamidin-Sepharose-Sätue entfernt werden, an die Thrombin spezifisch bindet. Andere Banden waren bei dem Versuch nicht erkennbar.

### Beispiel 8

### Herstellung von polyklonalen Kaninchen-Antiseren gegen hsGCα1 und hsGCβ1.

Antiseren wurden gewonnen durch Immunisierung von Kaninchen gegen synthetische Peptide, die der hsGCα1 (Phe-Thr-Pro-Arg-Ser-Arg-Glu-Glu-Leu-Pro-Pro-Asn-Phe-Pro (Abb. 22/SEQ IN NO: 5); Aminosäuren 634-647) bzw. der hsGCβ1 (Lys-Gly-Lys-Lys-Glu-Pro-Met-Gln-Val-Trp-Phe-Leu-Ser-Arg-Lys-Asn-Thr-Gly-Thr-Glu-Glu-Thr (Abb. 23/SEQ ID NO: 6); Aminosäuren 593-614) entsprechen, und die an KLH (Keyhole limpet hemocyanin) über einen zusätzlichen C-terminalen (α1) oder N-terminalen (β1) Cysteinrest gekoppelt wurden. Die Antiseren wurden affinitätsgereinigt mit den jeweiligen Peptiden, die an Epoxy-aktivierte Sepharose (Pharmacia, Freiburg) gekoppelt wurden (nach Anleitung des Herstellers).

### Beispiel 9

### Nachweis der hSGCα1 und hsGCβ1 in verschiedenen humanen Geweben im Immunoblot.

Humanes Lungengewebe wurde von einem tumorfreien Bereich einer Lungenresektion bezogen, humanes Cortex und Cerebellum von einer normalen Autopsie. Alle Gewebe waren sofort in flüssigem Stickstoff eingefroren und bei-70°C gelagert worden. Die gefrorenen Gewebe wurden gemörsert und das Pulver in 2 x konzentrierbem, vorerhitztem SDS-Stopp-Puffer (130 mM Tris-HCl pH 6,8 / 16 % [v/v] Glycerin / 4 % [w/v] SDS / 0,025 % [w/v] Bromphenolblau / 6,5 % [v/v] β-Mercaptoethanol) aufgenommen, 10 min bei 95°C inkubiert und dann 20 min bei 20000 x g zentrifugiert. Der Überstand wurde im Immunoblot eingesetzt (Antikörper siehe oben).

In allen drei Geweben kann eine Expression beider Untereinheiten (α1 und β1) nachgewiesen werden (Abb. 13). Keine Expression konnte dagegen in Niere, Leber und Pankreas gezeigt werden (Daten nicht gezeigt).

### Beispiel 10

### Konstruktion von rekombinanten adenoviralen hsGC-Vektoren

Die cDNAs für die hsGCα1 und hsGCβ1 wurden mit dem Restriktionsenzym EcoRI als Fragmente von 3,0 kb (hsGCα1) bzw. 2,4 kb (hsGCβ3) aus dem Originalplasmid herausgeschnitten. Diese Fragmente wurden jeweils in die EcoRI-Schnittstellen des adenoviralen Transferplasmids pZS2 inseriert (Abb. 16), das eine Adenovirus-Typ-5-Sequenz (Ad5) mit einer Deletion in der E1-Region (ΔE1) enthält, gefolgt von einer Expressionskassette mit CMV- (Cytomegalovirus) Promotor/-Enhancer und einer in diesem Plasmid einmaligen XbaI-Restriktions-Schnittstelle. XbaI-geschnittenes hsGCα1-pZS2 und hsGCβ1-pZS2 wurde in dieXbaI-Schnittstelle des langen Armes (RR5) von Ad5 inseriert (Abb. 16). Dies ergab die Adenovektoren Ad5CMVhsGCα1 bzw. Ad5CMVhsGCβ1. Die resultierenden rekombinanten Adenovirusvektoren sind replikationsdefizient, da ihnen der E1-Bereich fehlt. Zur Vermehrung der Viren wurden 293-Zellen, die E1 exprimieren, mit diesen Viren infiziert. Virale Plaques erschienen 12 bis 24 Stunden nach der Transfektion, und Viren aus Einzelplaques wurden nach einem Standardprotokoll aufgereinigt. Plaques, die rekombinantes Virus enthalten (Ad5CMVhsGCα1 oder Ad5CMVhsGCβ1) wurden durch PCR-Analyse identifiziert Das Plaqne-Maberial wurde dreimal eingefroren und aufgetaut, bei 37°C für 30 min in Lysepuffer (16,6 mM Ammoniumsulfat / 67 mM Tris-HCl pH 6,8 / 6,7 mM MgCl₂ / 5 mM β-Mercaptoethanol / 6,7 mM EDTA / 1,7 mM SDS / 50 µg/ml Proteinase K) inkubiert und dann für 10 min bei 85°C hitzeinaktiviert. Schließlich wurde die DNA mit einer Standard-Phenol/Chloroform-Extraktion aus dem Lysat isoliert und einer PCR-Analyse unterzogen.

### Beispiel 11

### Nachweis der cGMP-Bildung in EA.hy926-Zellen nach Koinfektion mit den hsGC-Adenovektoren Ad5CMVhsGCα1 und Ad5CMVhsGCβ1

Zehn 10-cm-Platten mit "EA.hy926"-Zellen wurden mit je 2 x 10¹⁰ pfu (Plaque-bildende Einheiten) pro Platte koinfiziert. Die Ernte erfolgte nach ca. 72 Stunden durch Zugabe von hypotonem Lysepuffer (25 mM Triethanolamin pH 7,8 / 1 mM EDTA / 5 mM DTT / 1 µM Leupeptin / 0,5 mg/l Trypsin-Inhibitor / 0,2 mM PMSF) und Ablösen mit einem Gummischaber. Das Homogenat wurde für 15 min bei 500 x g zentrifugiert und der Überstand mit einem gleichen Volumen Glycerin vermischt und bei -20°C gelagert Die hsGC-Stimulierbarkeit durch 100 µM SNP (Nitroprussidnatrium) wurde ermittelt durch Messung der cGMP-Konzentration und des cGMP-Gehaltes basal und nach SNP-Stimulation wie oben beschrieben [siehe Beispiel 5]. In drei Ansätzen (A, B, C) war im Vergleich zur Basalaktivität eine 7fache bis 10,75fach erhöhte cGMP-Konzentration nach SNP-Stimulation nachweisbar, während in der Kontrolle ohne Adenovirusinfektion keine signifikante Erhöhung zu messen war (Abb. 17).

### Beispiel 12

### Nachweis der cGMP-Bildung in ECV 304- und A10-Zellen nach Koinfektion mit den hsGC-Adenovektoren Ad5CMVhsGCα1 und Ad5CMVhsGCβ1

Je drei 10-cm Zellkulturschalen mit "ECV 304"-Zellen wurden mit 10⁹ - 5 x 10¹⁰ pfu (Plaque-bildende Einheiten) von Ad5CMVhsGCα1 bzw. Ad5CMVhsGCβ1 koinfiziert Die Ernte der Zellen erfolgte nach 72 Stunden durch 2-maliges Waschen der Zellen in PBS-Puffer (Phosphat-haltiger Kochsalzlösung), Zugabe von hypotonem Lysepuffer und weiterer Aufbereitung wie in Beispiel 11 beschrieben. Lagerung und Aktivitätsbestimmung (cGMP-Gehalt in pmol/mg/min) erfolgte wie in Beispiel 5 beschrieben. Nur nach Gentransfer von Ad5CMVhsGCα1und Ad5CMVhsGCβ1 (jeweils 5 x 10¹⁰ pfu) war eine signifikante Basalaktivität (ohne SNP-Zusatz) bzw. Maximalaktivität (mit Zusatz von 100 µM SNP zum Assay) nachweisbar (Abbildung 26). In Kontrollversuchen, ohne Ad5CMVhsGCα1 bzw. Ad5CMVhsGCβ1 war keine signifikante Erhöhung der cGMP-Bildung durch SNP nachweisbar. Qualitativ gleiche Daten wurden in "A10" glatte Blutgefäßmuskelzellen erhalten wenn diese ebenfalls mit 10⁹ - 5 x 10¹⁰ pfu Ad5CMVhsGCα1 bzw. Ad5CMVhsGCβ1 koinfiziert wurden. Auch hier war nur nach Gentransfer von Ad5CMVhsGCα1und Ad5CMVhsGCβ1 (jeweils 5 x 10¹⁰ pfu) eine signifikante Basalaktivität (ohne SNP-Zusatz) bzw. Maximalaktivität (mit Zusatz von 100 µM SNP zum Assay) nachweisbar (Abbildung 26). Auch hier war in Kontrollversuchen, ohne Ad5CMVhsGCα1 bzw. Ad5CMVhsGCβ1, keine signifikante Erhöhung der cGMP-Bildung durch SNP nachweisbar (Abbildung 28).

### Beispiel 13

### Kinetik der cGMP-Bildung in ECV 304- und A10-Zellen nach Koinfektion mit den hsGC-Adenovektoren Ad5CMVhsGCα1 und Ad5CMVhsGCβ1

Je drei 10-cm Zellkulturschalen mit "ECV 304"- bzw. "A10"-Zellen wurden mit 5 x 10¹⁰ pfu (Plaque-bildende Einheiten) von Ad5CMVhsGCα1 und Ad5CMVhsGCβ1 koinfiziert. Die Ernte der Zellen, weitere Aufbereitung, Lagerung und Aktivitätsbestimmung erfolgten wie in Beispiel 12, 11 und 5 beschrieben. Während an Tag 0 (also vor der Koinfektion) noch keine signifikante Erhöhung der cGMP-Bildung durch SNP nachweisbar war, war diese bis zu 15 Tage nach Gentransfer von Ad5CMVhsGCα1und Ad5CMVhsGCβ1 signifikant nachweisbar (Abbildung 28). Qualitativ gleiche Daten wurden in "A10" glatte Blutgefäßmuskelzellen erhalten wenn diese ebenfalls mit 5 x 10¹⁰ pfu Ad5CMVhsGCα1 und Ad5CMVhsGCβ1. koinfiziert wurden. Auch hier war eine cGMP-Bildung durch SNP noch 15 Tage nach Koinfektion nachweisbar (Abbildung 29). Auch hier war zum Tag 0 (also vor der Koinfektion) noch keine signifikante Erhöhung der cGMP-Bildung durch SNP nachweisbar. In den gleichen Proben waren nach adenoviralem Gentranfer sGC-Untereinheiten detektierbar, nicht aber in uninfizierten Kontrollzellen (Abbildung 30).

### Literatur:

Bradford, M.M. (1976) *Annal. Biochem.* 72: 248-254.

Friebe, A.; Schultz, G.; Koesling, D. (1996) *EMBO J*. **15**: 6863-6868.

Garthwaite, J.; Southam, E.; Boulton, C L; Nielsen, E. B.; Schmidt, K.; Mayer, B. (1995) *Mol*. *Pharmacol*. **48**:184-188.

Giuili, G.; Scholl, U.; Bulle, F.; Guellaen, G. (1992) *FEBS Lett*. **304**: 83-88.

Guthmann, F.; Mayer, B.; Koesling, D.; Kukovetz, W.R.; Boehme, E. (1992) *Naunyn-Schmiedeberg's Arch*. *Pharmacol*. **346:** 537-541.

Harteneck, C.; Wedel, B.; Koesling, D.; Malkewitz, J.; Böhme, E.; Schultz, G. (1991) *FEBS Lett*. **292**: 217-222.

Humbert, P.; Niroomand, F.; Fischer, G.; Mayer, B.; Koesling, D.; Hinsch, K.-D.; Gausepohl, H.; Frank, R.; Schultz, G.; Böhme, E. (1990) *Eur*. *J*. *Biochem*. **190**: 273-278.

Ko, F. N.; Wu, C. C.; Kuo, S.-C.; Lee, F.-Y.; Teng, CM. (1994) *Blood* **84:** 4226-4233.

Koesling,D.; Herz, J.; Gausepohl, H.; Niroomand, F.; Hinsch, K.-D.; Mülsch, A.; Böhme, E.; Schultz, G.; Frank, R. (1988) *FEBS Lett*. **239**: 29-34.

Schmidt, H. H. H. W.; Walter, U. (1994) *Cell* **78**: 919-925.

Schultz, G.; Böhme, E. (1984) erschienen in: Bergmeyer, H. U.; Bergmeyer, J.; Graßl, M. (Hrsg.): Methods of Enzymatic Analysis, VoL 4, Verlag Chemie, Weinheim, Seiten 379-389.

Wu, C.-C.; Ko, F.-N.; Kuo, S.-C.; Lee, F.-Y.; Teng, C.-M. (1995) *British J*. *Pharmacol*. **116**: 1973-1978.

## Patentansprüche

1. Isolierte und zur apparenten Homogenität gereinigte humane lösliche Guanylylcyclase α1/β1, wobei hsGCα1 identifiziert ist durch SEQ ID NO:2 und hsGCβ1 identifiziert ist durch SEQ ID NO:4.

2. Verfahren zur Herstellung der humanen löslichen Guanylylcyclase α1β1 nach Anspruch 1, umfassend die Expression eines die DNA-Sequenz für die Untereinheiten hsGCα1 und hsGCβ1 enthaltenden Expressionsvektors in prokaryontischen oder eukaryontischen Wirtszellen und Gewinnung der Guanylylcyclase.

3. Verfahren nach Anspruch 2, **dadurch gekennzeichnet, dass** der Schritt der Gewinnung der Guanylylcyclase α1/β1 eine Lyse der Zellen, die Affinitätschromatographie des Zell-Lysats und die anschließende Elution der Guanylylcyclase α1/β1 umfasst.

4. Verfahren nach Anspruch 2 oder 3, **dadurch gekennzeichnet, dass** der Expressionsvektor zusätzlich mindestens eine DNA-Sequenz für eine Domäne zur spezifischen Affinitätschromatographie (Affinitätstag) mit anhängender Proteaseschnittstelle enthält.

5. Verfahren nach Anspruch 4, **dadurch gekennzeichnet, dass** der Expressionsvektor die DNA-Sequenz für hsGCal mit Affinitätstag, die DNA-Sequenz für hsGCβ1 mit Affinitätstag, die DNA-Sequenz für hsGCα1 mit Affnitätstag und die DNA-Sequenz für hsGCβ1, die DNA-Sequenz für hsGCβ1 mit Affinitätstag und die DNA-Sequenz für hsGCα1 oder die DNA-Sequenz für hsGCα1 mit Affnitätstag und die DNA-Sequenz für hsGCβ1 mit Affinitätstag enthält.

6. Verfahren zur Herstellung der humanen löslichen Guanylylcyclase α1/β1 nach Anspruch 1, umfassend die getrennte Expression eines die DNA-Sequenz für hsGCα1 und hsGCβ1 enthaltenden Expressionsvektors in prokaryontischen oder eukaryontischen Wirtszellen, Gewinnung der Untereinheiten und Kombinieren der Untereinheiten hsGCα1 und hsGCβ1 zur dimeren Guanylylcyclase α1/β1.

7. Verfahren nach Anspruch 6, **dadurch gekennzeichnet**, das der Schritt der Gewinnung der Untereinheiten eine getrennte Lyse der hsGCα1 bzw. hsGCβ1 enthaltenden Zellen, die getrennte Affinitätschromatographie der Zell-Lysate und die anschließende Elution der Untereinheiten umfasst.

8. Verfahren zur Herstellung der humanen löslichen Guanylylcyclase α1/β1 nach Anspruch 1, umfassend die Koexpression der DNA-Sequenzen für hsGCα1 und hsGCβ1 in prokaryontischen oder eukaryontischen Wirtszellen, eine Lyse der hsGCα1 und hsGCβ1 enthaltenden Zellen, die Affinitätschromatographie und die anschließende Elution der löslichen Guanylylcyclase.

9. Verwendung der für die Untereinheiten hsGCα1 identifiziert durch SEQ ID NO:2 und hsGCβ1 identifiziert durch SEQ ID NO:4 der humanen löslichen Guanylylcyclase α1/β1 kodierenden Nukleinsäuresequenzen als Wirkstoff zur Herstellung eines Arzneimittels zur somatischen Gentherapie zur Prävention und Therapie von Artherosklerose und ihren Folgeerkrankungen, von Restenose, Ischämie (Infarkt,) peripheren arteriellen Verschlusskrankheiten und arterieller Hypertonie sowie zur Prävention bei Patienten mit Risikofaktoren für Atherosklerose, transitorische ischämische Attacken, zerebrale Ischämie, Schlaganfall (Apoplex), Koronare Herzkrankheit, Zustand nach koronarer Bypass-Operation, Carotisstenose, zur Verstärkung einer Therapie mit sGC-Aktivatoren, sGC-sensitivierenden Substanzen, Stickstoffmonoxid (NO-Donoren) oder Phosphodiesterase-Hemmstoffen, Herzinsuffizienz und Leberfunktionsstörungen.

10. Verwendung nach Anspruch 9, **dadurch gekennzeichnet, dass** als Wirkstoff ein Vektor verwendet wird, der die Nukleinsäuresequenzen für humane lösliche Guanylylcyclase α1 hsGCα1) und für humane lösliche Guanylylcyclase **β**1 (hsGCβ1) enthält.

11. Verwendung nach einem der Ansprüche 9 und 10, **dadurch gekennzeichnet, dass** der somatische Gentransfer erfolgt in Endothelzellen, glatte Blutgefäßmuskelzellen, Neointimazellen, Fibroblasten oder andere Blutgefäßzellen oder in die korpuskulären Blutbestandteile (Thrombozyten, Leukozyten und andere) oder in Leberzellen.

12. Monospezifische Antikörper gegen humane lösliche Guanylylcyclase α1/β1 nach Anspruch 1.

## Claims

1. An isolated human soluble guanylylcyclase α1/β1 purified to apparent homogeneity, wherein hsGCα1 is identified by SEQ ID NO:2 and hsGCβ1 is identified by SEQ ID NO:4.

2. A method for producing the human soluble guanylylcyclase α1/β1 according to claim 1, comprising expression of an expression vector containing the DNA sequence for the subunits hsGCα1 and hsGCβ1 in prokaryotic or eukaryotic host cells, and recovery of the guanylylcyclase.

3. The method according to claim 2, **characterized in that** the step of recovering the guanylylcyclase α1/β1 comprises lysis of the cells, affinity chromatography of the cell lysate, and subsequent elution of the guanylylcyclase α1/β1.

4. The method according to claim 2 or 3, **characterized in that** the expression vector additionally contains at least one DNA sequence for a specific affinity chromatography domain (affinity tag) with an adherent protease cleavage site.

5. The method according to claim 4, **characterized in that** the expression vector contains the DNA sequence for hsGCα1 with affinity tag, the DNA sequence for hsGCβ1 with affinity tag, the DNA sequence for hsGCα1 with affinity tag and the DNA sequence for hsGCβ1, the DNA sequence for hsGCβ1 with affinity tag and the DNA sequence for hsGCα1, or the DNA sequence for hsGCα1 with affinity tag and the DNA sequence for hsGCβ1 with affinity tag.

6. A method for producing the human soluble guanylylcyclase α1/β1 according to claim 1, comprising the separate expression of an expression vector containing the DNA sequence for hsGCα1 and hsGCβ1 in prokaryotic or eukaryotic host cells, recovery of the subunits, and combination of the subunits hsGCα1 and hsGCβ1 to dimeric guanylylcyclase α1/β1.

7. The method according to claim 6, **characterized in that** the step of recovering the subunits comprises separate lysis of cells containing hsGCα1 and hsGCβ1, separate affinity chromatography of the cell lysates, and subsequent elution of the subunits.

8. A method for producing the human soluble guanylylcyclase α1/β1 according to claim 1, comprising co-expression of the DNA sequences for hsGCα1 and hsGCβ1 in prokaryotic and eukaryotic host cells, lysis of cells containing hsGCα1 and hsGCβ1, affinity chromatography, and subsequent elution of soluble guanylylcyclase.

9. The use of nucleic acid sequences encoding subunits hsGCα1 identified by SEQ ID NO:2 and hsGCβ1 identified by SEQ ID NO:4 of human soluble guanylylcyclase α1/β1 as therapeutic agents for the preparation of a pharmaceutical composition for somatic gene therapy for prevention and therapy of atherosclerosis and resulting diseases, of restenosis, ischaemia (infarct), peripheral arterial obstructive disease, and arterial hypertension, as well as for prevention in patients with risk factors for atherosclerosis, transient ischaemic attacks, cerebral ischaemia, apoplectic stroke (apoplexy), coronary heart disease, condition after coronary bypass surgery, carotid stenosis, for reinforcing therapy with sGC activators, sGC sensitizing substances, nitrogen monoxide (NO donors), or phosphodiesterase inhibitors, cardiac insufficiency, and liver dysfunction.

10. The use according to claim 9, **characterized in that** a vector is used as therapeutic agent, which contains the nucleic acid sequences for human soluble guanylylcyclase α1 (hsGCα1) and human soluble guanylylcyclase β1 (hsGCβ1).

11. The use according to any one of claims 9 and 10, **characterized in that** the somatic gene transfer occurs in endothelial cells, smooth blood vessel cells, neointima cells, fibroblasts, or other blood vessel cells, or in the corpuscular components of the blood (platelets, leukocytes and others), or in liver cells.

12. A monospecific antibody against human soluble guanylylcyclase α1/β1 according to claim 1.

## Revendications

1. Guanylylcyclase α1/β1 humaine soluble isolée et purifiée jusqu'à homogénéité apparente, dans laquelle hsGCα1 est identifiée par SEQ ID NO:2 et hsGCβ1 est identifiée par SEQ ID NO:4.

2. Procédé pour la préparation de la guanylylcyclase α1β1 humaine soluble selon la revendication 1, comprenant l'expression d'un vecteur d'expression contenant la séquence d'ADN pour les sous-unités hsGCα1 et hsGCβ1 dans des cellules hôtes procaryotes ou eucaryotes et l'obtention de la guanylylcyclase.

3. Procédé selon la revendication 2, **caractérisé en ce que** l'étape d'obtention de la guanylylcyclase α1/β1 comprend une lyse des cellules, la chromatographie d'affinité du lysat cellulaire et ensuite l'élution de la guanylylcyclase α1/β1.

4. Procédé selon la revendication 2 ou 3, **caractérisé en ce que** le vecteur d'expression comprend de plus au moins une séquence d'ADN pour un domaine pour la chromatographie d'affinité spécifique (marqueur d'affinité) avec une position de restriction de protéase pendante.

5. Procédé selon la revendication 4, **caractérisé en ce que** le vecteur d'expression contient la séquence d'ADN pour hsGCα1 avec un marqueur d'affinité, la séquence d'ADN pour hsGCβ1 avec un marqueur d'affinité, la séquence d'ADN pour hsGCα1 avec un marqueur d'affinité et la séquence d'ADN pour hsGCβ1, la séquence d'ADN pour hsGCβ1 avec un marqueur d'affinité et la séquence d'ADN pour hsGCα1 ou la séquence d'ADN pour hsGCα1 avec un marqueur d'affinité et la séquence d'ADN pour hsGCβ1 avec un marqueur d'affinité.

6. Procédé pour la préparation de la guanylylcyclase α1/β1 humaine soluble selon la revendication 1, comprenant l'expression séparée d'un vecteur d'expression contenant la séquence d'ADN pour hsGCα1 et hsGCβ1 dans des cellules hôtes procaryotes ou eucaryotes, l'obtention des sous-unités et la combinaison des sous-unités hsGCα1 et hsGCβ1 pour donner la guanylylcyclase α1/β1 dimère.

7. Procédé selon la revendication 6, **caractérisé en ce que** l'étape de l'obtention des sous-unités comprend une lyse séparée des cellules contenant hsGCα1 et hsGCβ1, la chromatographie d'affinité séparée des lysats cellulaires et ensuite l'élution des sous-unités.

8. Procédé pour la préparation de la guanylylcyclase α1/β1 humaine soluble selon la revendication 1, comprenant la co-expression des séquences d'ADN pour hsGCα1 et hsGCβ1 dans des cellules hôtes procaryotes ou eucaryotes, une lyse des cellules contenant hsGCα1 et hsGCβ1, la chromatographie d'affinité et ensuite l'élution de la guanylylcyclase soluble.

9. Utilisation des séquences d'acide nucléique codant pour les sous-unités hsGCα1 identifiées par SEQ ID NO: 2 et hsGCβ1 identifiées par SEQ ID NO: 4 de la guanylylcyclase α1/β1 humaine soluble comme substance active pour la préparation d'un médicament pour la thérapie génique somatique destinée à la prévention et la thérapie de l'athérosclérose et des maladies qui en découlent, la resténose, l'ischémie (infarctus), les maladies d'occlusion des artères périphériques et l'hypertonie artérielle ainsi que pour la prévention chez les patients avec des facteurs de risque pour l'athérosclérose, les attaques ischémiques transitoires, l'ischémie cérébrale, l'apoplexie, la maladie cardiaque coronarienne, l'état après une opération de pontage coronarien, la sténose de la carotide, pour le renforcement d'une thérapie avec des activateurs de sGC, des substances sensibilisatrices à sGC, le monoxyde d'azote (donneurs de NO) ou les substances inhibitrices de phosphodiestérase, l'insuffisance cardiaque et les troubles hépatiques fonctionnels.

10. Utilisation selon la revendication 9, **caractérisée en ce qu'**on utilise comme substance active un vecteur qui contient les séquences d'acides nucléiques pour la guanylylcyclase humaine soluble α1 (hsGCα1) et pour la guanylylcyclase humaine soluble β1 (hsGCβ1).

11. Utilisation selon une des revendications 9 et 10, **caractérisée en ce qu'**on réalise le transfert somatique de gènes dans des cellules endothéliales, des cellules des muscles des vaisseaux sanguins, des cellules de néo-intima, des fibroblastes ou d'autres cellules de vaisseaux sanguins ou dans les constituants corpusculaires du sang (thrombocytes, leucocytes et autres) ou dans des cellules hépatiques.

12. Anticorps mono-spécifiques contre la guanylylcyclase α1/β1 humaine soluble selon la revendication 1.


### SEQUENZPROTOKOLL

(1) ALLGEMEINE ANGABEN:
   (i) ANMELDER:
      (A) NAME: Vasopharm Biotech GmbH & Co. KG
      (B) STRASSE: Leichtackerstr. 6
      (C) ORT: Veitshöchheim
      (D) BUNDESLAND: Bayern
      (E) LAND: Deutschland
      (F) POSTLEITZAHL: 97209
      (G) TELEFON:
   (ii) BEZEICHNUNG DER ERFINDUNG: Isolierte und gereinigte humane lösliche Guanylylcyclase α1/β1 (hsGCα1/β1)
   (iii) ANZAHL DER SEQUENZEN: 10
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: Patentin Release #1.0, Version #1.30 (EPA)
(2) ANGABEN ZU SEQ ID NO: 1:
   (i) SEQUENZKENNZEICHEN:
      (A) LANGE: 3015 Basenpaare
      (B) ART: Nucleotid
      (C) STRANGFORM: Doppelstrang
      (D) TOPOLOGIE: linear
   (ii) ART DES MOLEKÜLS: Genom-DNA
   (xi) SEQUENZBESCHREIBUNG: SEQ ID NO:1:
   CCCTTATGGC GATTGGGCGG CTGCAGAGAC CAGGACTCAG TTCCCCTGCC CTAGTCTGAG 60
   CCTAGTGGGT GGGACTCAGC TCAGAGTCAG TTTTCCAGAA GCAGGTTTCA GTGCAGAGTT 120
   TTCCTACACT TTTCCTGCGC TAGAGCAGCG AGCAGCCTGG AACAGACCCA GGCGGAGGAC 180
   ACCTGTGGGG GAGGGAGCGC CTGGAGGAGC TTAGAGACCC CAGCCGGGCG TGATCTCACC 240
   ATGTGCGGAT TTGCGAGGCG CGCCCTGGAG CTGCTAGAGA TCCGGAAGCA CAGCCCCGAG 300
   GTGTGCGAAG CCACCAAGAC TGCGGCTCTT GGAGAAAGCG TGAGCAGGGG GCCACCGCGG 360
   TCTCCGCGCC TGTCTGCACC CTGTCGCCTG AGCTGCCTGA CAGTGACAAT GACATCCCAG 420
   TTACCAGTGT CCTTGAATTG ATAGTGGCTT CTGTTTGTCA GTCTCATATA AGAACTACAG 480
   CTCATCAGGA GGAGATCGCA GCAGGGTAAG AGACACCAAC ACCATGTTCT GCACGAAGCT 540
   CAAGGATCTC AAGATCACAG GAGAGTGTCC TTTCTCCTTA CTGGCACCAG GTCAAGTTCC 600
   TAACGAGTCT TCAGAGGAGG CAGCAGGAAG CTCAGAGAGC TGCAAAGCAA CCGTGCCCAT 660
   CTGTCAAGAC ATTCCTGAGA AGAACATACA AGAAAGTCTT CCTCAAAGAA AAACCAGTCG 720
   GAGCCGAGTC TATCTTCACA CTTTGGCAGA GAGTATTTGC AAACTGATTT TCCCAGAGTT 780
   TGAACGGCTG AATGTTGCAC TTCAGAGAAC ATTGGCAAAG CACAAAATAA AAGAAAGCAG 840
   GAAATCTTTG GAAAGAGAAG ACTTTGAAAA AACAATTGCA GAGCAAGCAG TTGCAGCAGG 900
   AGTTCCAGTG GAGGTTATCA AAGAATCTCT TGGTGAAGAG GTTTTTAAAA TATGTTACGA 960
   GGAAGATGAA AACATCCTTG GGGTGGTTGG AGGCACCCTT AAAGATTTTT TAAACAGCTT 1020
   CAGTACCCTT CTGAAACAGA GCAGCCATTG CCAAGAAGCA GGAAAAAGGG GCAGGCTTGA 1080
   GGACGCCTCC ATTCTATGCC TGGATAAGGA GGATGATTTT CTACATGTTT ACTACTTCTT 1140
   CCCTAAGAGA ACCACCTCCC TGATTCTTCC CGGCATCATA AAGGCAGCTG CTCACGTATT 1200
   ATATGAAACG GAAGTGGAAG TGTCGTTAAT GCCTCCCTGC TTCCATAATG ATTGCAGCGA 1260
   GTTTGTGAAT CAGCCCTACT TGTTGTACTC CGTTCACATG AAAAGCACCA AGCCATCCCT 1320
   GTCCCCCAGC AAACCCCAGT CCTCGCTGGT GATTCCCACA TCGCTATTCT GCAAGACATT 1380
   TCCATTCCAT TTCATGTTTG ACAAAGATAT GACAATTCTG CAATTTGGCA ATGGCATCAG 1440
   AAGGCTGATG AACAGGAGAG ACTTTCAAGG AAAGCCTAAT TTTGAAGAAT ACTTTGAAAT 1500
   TCTGACTCCA AAAATCAACC AGACGTTTAG CGGGATCATG ACTATGTTGA ATATGCAGTT 1560
   TGTTGTACGA GTGAGGAGAT GGGACAACTC TGTGAAGAAA TCTTCAAGGG TTATGGACCT 1620
   CAAAGGCCAA ATGATCTACA TTGTTGAATC CAGTGCAATC TTGTTTTTGG GGTCACCCTG 1680
   TGTGGACAGA TTAGAAGATT TTACAGGACG AGGGCTCTAC CTCTCAGACA TCCCAATTCA 1740
   CAATGCACTG AGGGATGTGG TCTTAATAGG GGAACAAGCC CGAGCTCAAG ATGGCCTGAA 1800
   GAAGAGGCTG GGGAAGCTGA AGGCTACCCT TGAGCAAGCC CACCAAGCCC TGGAGGAGGA 1860
   GAAGAAAAAG ACAGTAGACC TTCTGTGCTC CATATTTCCC TGTGAGGTTG CTCAGCAGCT 1920
   GTGGCAAGGG CAAGTTGTGC AAGCCAAGAA GTTCAGTAAT GTCACCATGC TCTTCTCAGA 1980
   CATCGTTGGG TTCACTGCCA TCTGCTCCCA GTGCTCACCG CTGCAGGTCA TCACCATGCT 2040
   CAATGCACTG TACACTCGCT TCGACCAGCA GTGTGGAGAG CTGGATGTCT ACAAGGTGGA 2100
   GACCATTGGC GATGCCTATT GTGTAGCTGG GGGATTACAC AAAGAGAGTG ATACTCATGC 2160
   TGTTCAGATA GCGCTGATGG CCCTGAAGAT GATGGAGCTC TCTGATGAAG TTATGTCTCC 2220
   CCATGGAGAA CCTATCAAGA TGCGAATTGG ACTGCACTCT GGATCAGTTT TTGCTGGCGT 2280
   CGTTGGAGTT AAAATGCCCC GTTACTGTCT TTTTGGAAAC AATGTCACTC TGGCTAACAA 2340
   ATTTGAGTCC TGCAGTGTAC CACGAAAAAT CAATGTCAGC CCAACAACTT ACAGATTACT 2400
   CAAAGACTGT CCTGGTTTCG TGTTTACCCC TCGATCAAGG GAGGAACTTC CACCAAACTT 2460
   CCCTAGTGAA ATCCCCGGAA TCTGCCATTT TCTGGATGCT TACCAACAAG GAACAAACTC 2520
   AAAACCATGC TTCCAAAAGA AAGATGTGGA AGATGGCAAT GCCAATTTTT TAGGCAAAGC 2580
   ATCAGGAATA GATTAGCAAC CTATATACCT ATTTATAAGT CTTTGGGGTT TGACTCATTG 2640
   AAGATGTGTA GAGCCTCTGA AAGCACTTTA GGGATTGTAG ATGGCTAACA AGCAGTATTA 2700
   AAATTTCAGG AGCCAAGTCA CAATCTTTCT CCTGTTTAAC ATGACAAAAT GTACTCACTT 2760
   CAGTACTTCA GCTCTTCAAG AAAAAAAAAA AAACCTTAAA AAGCTACTTT TGTGGGAGTA 2820
   TTTCTATTAT ATAACCAGCA CTTACTACCT GTACTCAAAA TTCAGCACCT TGTACATATA 2880
   TCAGATAATT GTAGTCAATT GTACAAACTG ATGGAGTCAC CTGCAATCTC ATATCCTGGT 2940
   GGAATGCCAT GGTTATTAAA GTGTGTTTGT GATAGTGTCG TCAAAAAAAA AAAAAAAAAA 3000
   AAAAAAAAAA AAAAA 3015
(2) ANGABEN ZU SEQ ID NO: 2:
   (i) SEQUENZKENNZEICHEN:
      (A) LÄHGE: 695 Aminosäuren
      (B) ART: Aminosäure
      (D) TOPOLOGIE: linear
   (ii) ART DES MOLEKUELS: Protein (humanen löslichen Guanylylcyclase a1 (hsGCa1))
   (xi) SEQUENZBESCHREIBUNG: SEQ ID NO: 2:
(2) ANGABEN ZU SEQ ID NO: 3:
   (i) SEQUENZKENNZEICHEN:
      (A) LANGE: 2443 Basenpaare
      (B) ART: Nucleotid
      (C) STRANGFORM: Doppelstrang
      (D) TOPOLOGIE: linear
   (ii) ART DES MOLEKÜLS: Genom-DNA
   (xi) SEQUENZBESCHREIBUNG: SEQ ID NO:3:
   CCCCCCCCCG CCGCTGCCGC CTCTGCCTGG GTCCCTTCGG CCGTACCTCT GCGTGGGGGC     60
   TGCCTCCCCG GCTCCCGGTG CAGACACCAT GTACGGATTT GTGAATCACG CCCTGGAGTT    120
   GCTGGTGATC CGCAATTACG GCCCCGAGGT GTGGGAAGAC ATCAAAAAAG AGGCACAGTT    180
   AGATGAAGAA GGACAGTTTC TTGTCAGAAT AATATATGAT GACTCCAAAA CTTATGATTT    240
   GGTTGCTGCT GCAAGCAAAG TCCTCAATCT CAATGCTGGA GAAATCCTCC AAATGTTTGG    300
   GAAGATGTTT TTCGTCTTTT GCCAAGAATC TGGTTATGAT ACAATCTTGC GTGTCCTGGG    360
   CTCTAATGTC AGAGAATTTC TACAGAACCT TGATGCTCTG CACGACCACC TTGCTACCAT    420
   CTACCCAGGA ATGCGTGCAC CTTCCTTTAG GTGCACTGAT GCAGAAAAGG GCAAAGGACT    480
   CATTTTGCAC TACTACTCAG AGAGAGAAGG ACTTCAGGAT ATTGTCATTG GAATCATCAA    540
   AACAGTGGCA CAACAAATCC ATGGCACTGA AATAGACATG AAGGTTATTC AGCAAAGAAA    600
   TGAAGAATGT GATCATACTC AATTTTTAAT TGAAGAAAAA GAGTCAAAAG AAGAGGATTT    660
   TTATGAAGAT CTTGACAGAT TTGAAGAAAA TGGTACCCAG GAATCACGCA TCAGCCCATA    720
   TACATTCTGC AAAGCTTTTC CTTTTCATAT AATATTTGAC CGGGACCTAG TGGTCACTCA    780
   GTGTGGCAAT GCTATATACA GAGTTCTCCC CCAGCTCCAG CCTGGGAATT GCAGCCTTCT    840
   GTCTGTCTTC TCGCTGGTTC GTCCTCATAT TGATATTAGT TTCCATGGGA TCCTTTCTCA    900
   CATCAATACT GTTTTTGTAT TGAGAAGCAA GGAAGGATTG TTGGATGTGG AGAAATTAGA    960
   ATGTGAGGAT GAACTGACTG GGACTGAGAT CAGCTGCTTA CGTCTCAAGG GTCAAATGAT   1020
   CTACTTACCT GAAGCAGATA GCATACTTTT TCTATGTTCA CCAAGTGTCA TGAACCTGGA   1080
   CGATTTGACA AGGAGAGGGC TGTATCTAAG TGACATCCCT CTGCATGATG CCACGCGCGA   1140
   TCTTGTTCTT TTGGGAGAAC AATTTAGAGA GGAATACAAA CTCACCCAAG AACTGGAAAT   1200
   CCTCACTGAC AGGCTACAGC TCACGTTAAG AGCCCTGGAA GATGAAAAGA AAAAGACAGA   1260
   CACATTGCTG TATTCTGTCC TTCCTCCGTC TGTTGCCAAT GAGCTGCGGC ACAAGCGTCC   1320
   AGTGCCTGCC AAAAGATATG ACAATGTGAC CATCCTCTTT AGTGGCATTG TGGGCTTCAA   1380
   TGCTTTCTGT AGCAAGCATG CATCTGGAGA AGGAGCCATG AAGATCGTCA ACCTCCTCAA   1440
   CGACCTCTAC ACCAGATTTG ACACACTGAC TGATTCCCGG AAAAACCCAT TTGTTTATAA   1500
   GGTGGAGACT GTTGGTGACA AGTATATGAC AGTGAGTGGT TTACCAGAGC CATGCATTCA   1560
   CCATGCACGA TCCATCTGCC ACCTGGCCTT GGACATGATG GAAATTGCTG GCCAGGTTCA   1620
   AGTAGATGGT GAATCTGTTC AGATAACAAT AGGGATACAC ACTGGAGAGG TAGTTACAGG   1680
   TGTCATAGGA CAGCGGATGC CTCGATACTG TCTTTTTGGG AATACTGTCA ACCTCACAAG   1740
   CCGAACAGAA ACCACAGGAG AAAAGGGAAA AATAAATGTG TCTGAATATA CATACAGATG   1800
   TCTTATGTCT CCAGAAAATT CAGATCCACA ATTCCACTTG GAGCACAGAG GCCCAGTGTC   1860
   CATGAAGGGC AAAAAAGAAC CAATGCAAGT TTGGTTTCTA TCCAGAAAAA ATACAGGAAC   1920
   AGAGGAAACA AAGCAGGATG ATGACTGAAT CTTGGATTAT GGGGTGAAGA GGAGTACAGA   1980
   CTAGGTTCCA GTTTTCTCCT AACACGTGCC AAGCCCAGGA GCAGTTCTTC CCTATGGATA   2040
   CAGATTTTCT TTTGTCCTTG TCCATTACCC CAAGACTTTC TTCTAGATAT ATCTCTCACT   2100
   ATCCGTTATT CAACCTTAGC TCTGCTTTCT ATTACTTTTT AGGCTTTAGT ATATTATCTA   2160
   AAGTTTGGCT TTTGATGTGG ATGATGTGAG CTTCATGTGT CTTAAAATCT ACTACAAGCA   2220
   TTACCTAACA TGGTGATCTG CAAGTAGTAG GCACCCAATA AATATTTGTT GAATTTAGTT   2280
   AAATGAAACT GAACAGTGTT TGGCCATGTG TATATTTATA TCATGTTTAC CAAATCTGTT   2340
   TAGTGTTCCA CATATATGTA TATGTATATT TTAATGACTA TAATGTAATA AAGTTTATAT   2400
   CATGTTGGTG TATATCATTA TAGAAATCAT TTTCTAAAGG AGT                     2443
(2) ANGABEN ZU SEQ ID NO: 4:
   (i) SEQUENZKENNZEICHEN:
      (A) LÄNGE: 619 Aminosäuren
      (B) ART: Aminosäure
      (D) TOPOLOGIE: linear
   (ii) ART DES MOLEKUELS: Protein (humanen löslichen Guanylylcyclase bl (hsGCb1))
   (xi) SEQUENZBESCHREIBUNG: SEQ ID NO: 4:
   Met Tyr Gly Phe Val Asn His Ala Leu Glu Leu Leu Val Ile Arg Asn
                       5                   10                  15
   Tyr Gly Pro Glu Val Trp Glu Asp Ile Lys Lys Glu Ala Gln Leu Asp
               20                  25                  30
   Glu Glu Gly Gln Phe Leu Val Arg Ile Ile Tyr Asp Asp Ser Lys Thr
           35                  40                  45
   Tyr Asp Leu Val Ala Ala Ala Ser Lys Val Leu Asn Leu Asn Ala Gly
       50                  55                  60
   Glu Ile Leu Gln Met Phe Gly Lys Met Phe Phe Val Phe Cys Gln Glu
   65                  70                  75                  80
   Ser Gly Tyr Asp Thr Ile Leu Arg Val Leu Gly Ser Asn Val Arg Glu
                   85                  90                  95
   Phe Leu Gln Asn Leu Asp Ala Leu His Asp His Leu Ala Thr Ile Tyr
               100                 105                 110
   Pro Gly Met Arg Ala Pro Ser Phe Arg Cys Thr Asp Ala Glu Lys Gly
           115                 120                 125
   Lys Gly Leu Ile Leu His Tyr Tyr Ser Glu Arg Glu Gly Leu Gln Asp
       130                 135                 140
   Ile Val Ile Gly Ile Ile Lys Thr Val Ala Gln Gln Ile His Gly Thr
   145                 150                 155                 160
   Glu Ile Asp Met Lys Val Ile Gln Gln Arg Asn Glu Glu Cys Asp His
                   165                 170                 175
   Thr Gln Phe Leu Ile Glu Glu Lys Glu Ser Lys Glu Glu Asp Phe Tyr
               180                 185                 190
   Glu Asp Leu Asp Arg Phe Glu Glu Asn Gly Thr Gln Glu Ser Arg Ile
           195                 200                 205
   Ser Pro Tyr Thr Phe Cys Lys Ala Phe Pro Phe His Ile Ile Phe Asp
       210                 215                 220
   Arg Asp Leu Val Val Thr Gln Cys Gly Asn Ala Ile Tyr Arg Val Leu
   225                 230                 235                 240
   Pro Gln Leu Gln Pro Gly Asn Cys Ser Leu Leu Ser Val Phe Ser Leu
                   245                 250                 255
   Val Arg Pro His Ile Asp Ile Ser Phe His Gly Ile Leu Ser His Ile
               260                 265                 270
   Asn Thr Val Phe Val Leu Arg Ser Lys Glu Gly Leu Leu Asp Val Glu
           275                 280                 285
   Lys Leu Glu Cys Glu Asp Glu Leu Thr Gly Thr Glu Ile Ser Cys Leu
       290                 295                 300
   Arg Leu Lys Gly Gln Met Ile Tyr Leu Pro Glu Ala Asp Ser Ile Leu
   305                 310                 315                 320
   Phe Leu Cys Ser Pro Ser Val Met Asn Leu Asp Asp Leu Thr Arg Arg
                   325                 330                 335
   Gly Leu Tyr Leu Ser Asp Ile Pro Leu His Asp Ala Thr Arg Asp Leu
               340                 345                 350
   Val Leu Leu Gly Glu Gln Phe Arg Glu Glu Tyr Lys Leu Thr Gln Glu
           355                 360                 365
   Leu Glu Ile Leu Thr Asp Arg Leu Gln Leu Thr Leu Arg Ala Leu Glu
       370                 375                 380
   Asp Glu Lys Lys Lys Thr Asp Thr Leu Leu Tyr Ser Val Leu Pro Pro
   385                 390                 395                 400
   Ser Val Ala Asn Glu Leu Arg His Lys Arg Pro Val Pro Ala Lys Arg
                   405                 410                 415
   Tyr Asp Asn Val Thr Ile Leu Phe Ser Gly Ile Val Gly Phe Asn Ala
               420                 425                 430
   Phe Cys Ser Lys His Ala Ser Gly Glu Gly Ala Met Lys Ile Val Asn
           435                 440                 445
   Leu Leu Asn Asp Leu Tyr Thr Arg Phe Asp Thr Leu Thr Asp Ser Arg
       450                 455                 460
   Lys Asn Pro Phe Val Tyr Lys Val Glu Thr Val Gly Asp Lys Tyr Met
   465                 470                 475                 480
   Thr Val Ser Gly Leu Pro Glu Pro Cys Ile His His Ala Arg Ser Ile
                   485                 490                 495
   Cys His Leu Ala Leu Asp Met Met Glu Ile Ala Gly Gln Val Gln Val
               500                 505                 510
   Asp Gly Glu Ser Val Gln Ile Thr Ile Gly Ile His Thr Gly Glu Val
           515                 520                 525
   Val Thr Gly Val Ile Gly Gln Arg Met Pro Arg Tyr Cys Leu Phe Gly
       530                 535                 540
   Asn Thr Val Asn Leu Thr Ser Arg Thr Glu Thr Thr Gly Glu Lys Gly
   545                 550                 555                 560
   Lys Ile Asn Val Ser Glu Tyr Thr Tyr Arg Cys Leu Met Ser Pro Glu
                   565                 570                 575
   Asn Ser Asp Pro Gln Phe His Leu Glu His Arg Gly Pro Val Ser Met
               580                 585                 590
   Lys Gly Lys Lys Glu Pro Met Gln Val Trp Phe Leu Ser Arg Lys Asn
   595 600 605
   Thr Gly Thr Glu Glu Thr Lys Gln Asp Asp Asp
   610 615
(2) ANGABEN ZU SEQ ID NO: 5:
   (i) SEQUENZKENNZEICHEN:
      (A) LÄNGE: 14 Aminosäuren
      (B) ART: Aminosäure
      (D) TOPOLOGIE: linear
   (ii) ART DES MOLEKUELS: Peptid (Aminosäuren 634-647 der hsGCa1)
   (xi) SEQUENZBESCHREIBUNG: SEQ ID NO: 5:
   Phe Thr Pro Arg Ser Arg Glu Glu Leu Pro Pro Asn Phe Pro
   5 10
(2) ANGABEN ZU SEQ ID NO: 6:
   (i) SEQUENZKENNZEICHEN:
      (A) LÄNGE: 22 Aminosäuren
      (B) ART: Aminosäure
      (D) TOPOLOGIE: linear
   (ii) ART DES MOLEKUELS: Peptid (Aminosäuren 593-614 der hsGCb1)
   (xi) SEQUENZBESCHREIBUNG: SEQ ID NO: 6:
   Lys Gly Lys Lys Glu Pro Met Gln Val Trp Phe Leu Ser Arg Lys Asn
   5 10 15
   Thr Gly Thr Glu Glu Thr
   20
(2) ANGABEN ZU SEQ ID NO: 7:
   (i) SEQUENZKENNZEICHEN:
      (A) LÄNGE: 28 Basenpaare
      (B) ART: Nucleotid
      (C) STRANGFORM: Einzelstrang
      (D) TOPOLOGIE: linear
   (ii) ART DES MOLEKULS: Genom-DNA
   (iii) HYPOTHETISCH: Nein
   (iv) ANTISENSE: Nein
   (xi) SEQUENZBESCHREIBUNG: SEQ ID NO:7:
   AAAAGGATCC ATGTTCTGCA CGAAGCTC 28
(2) ANGABEN ZU SEQ ID NO: 8:
   (i) SEQUENZKENNZEICHEN:
      (A) LÄNGE: 18 Basenpaare
      (B) ART: Nucleotid
      (C) STRANGFORM: Einzelstrang
      (D) TOPOLOGIE: linear
   (ii) ART DES MOLEKÜLS: Genom-DNA
   (iii) HYPOTHETISCH: Mein
   (iv) ANTISENSE: Nein
   (xi) SEQUENZBESCHREIBUNG: SEQ ID NO:8:
   ATTATGGAAG CACGGAGG 18
(2) ANGABEN ZU SEQ ID NO: 9:
   (i) SEQUENZKENNZEICHEN:
      (A) LANGE: 28 Basenpaare
      (B) ART: Nucleotid
      (C) STRANGFORM: Einzelstrang
      (D) TOPOLOGIE: linear
   (ii) ART DES MOLEKÜLS: Genom-DNA
   (iii) HYPOTHETISCH: Nein
   (iv) ANTISENSE: Nein
   (xi) SEQUENZBESCHREIBUNG: SEQ ID NO:9:
   AAAAGGATCC ATGTACGGAT TTGTGAAT 28
(2) ANGABEN ZU SEQ ID NO: 10:
   (i) SEQUENZKENNZEICHEN:
      (A) LÄNGE: 20 Basenpaare
      (B) ART: Nucleotid
      (C) STRANGFORM: Einzelstrang
      (D) TOPOLOGIE: linear
   (ii) ART DES MOLEKÜLS: Genom-DNA
   (iii) HYPOTHETISCH: Kein
   (iv) ANTISENSE: Nein
   (xi) SEQUENZBESCHREIBUNG: SEQ ID NO:10:
   ATGCGTGATT CCTGGGTACC 20